# EUROPEAN PATENT SPECIFICATION

(11) **EP 2 559 760 B1**
(45) Date of publication and mention of the grant of the patent: **21.10.2015**
(21) Application number: 11750394.6
(22) Date of filing: 04.03.2011
(51) Int. Cl.: C12N 15/09, C07K 14/47

(54) **Collectin fragment for the treatment of vascular disease**
Collectin Fragment zur linderung von Gefässkrankheiten
Fragment d'une Collectine pour l'AMÉLIORATION des maladies vasculaires

(30) Priority: 04.03.2010 JP 2010047335
(43) Date of publication of application: 20.02.2013
(73) Proprietor: Fuso Pharmaceutical Industries, Ltd., Osaka-shi, Osaka 541-0045 (JP)
(72) Inventor: WAKAMIYA, Nobutaka, Asahikawa-shi, Hokkaido 078-8345 (JP); OTANI, Katsuki, Asahikawa-shi, Hokkaido 078-8371 (JP)
(74) Representative: Price, Phillip
(86) International application number: PCT/JP2011/001285
(87) International publication number: WO 2011/108281

(56) References cited:
- WO-A1-00/11161
- WO-A1-01/59107
- WO-A2-00/68380
- WO-A2-2004/024925
- JP-A- 2001 340 089
- OHTANI K ET AL.: 'The membrane-type collectin CL-P1 is a scavenger receptor on vascular endothelial cells' J BIOL CHEM. vol. 276, no. 47, 2001, pages 44222 - 44228
- DATABASE DATABASE DDBJ/EMBL/GENBAN [Online] 26 January 2010 Database accession no. 001020892

## Description

### [TECHNICAL FIELD]

The present invention involves in a pathogenic mechanism of ischemic disease and, in particular, relates to a polypeptide which is useful to develop a reagent and medicine that are preferable for preventing and treating arteriosclerosis.

### [BACKGROUND ART]

According to recent cause-specific death number in Japan, death number due to angiopathy still accounts for the larger proportion and is substantially equivalent to those due to cancers. Of these angiopathys, disease involved directly or indirectly with arteriosclerosis dominated them. Of diseases designated as "arteriosclerosis" involved with the symptom of arterial, in general, they include angiopathy like aortic incompetence, renal hypertension, ischemic cardiac disease, stroke, arteriosclerosis obliterans, aneurism, arterial dissection and acute arterial obstruction.

When symptom of such arteriosclerosis is progressed, disintegration of vascular wall and obstruction of blood vessel are realized. It was thought from previous study that condition of such arteriosclerosis would be progressed with dysfunction of endothelial cells, lipid deposition in vascular wall and following accumulation of monocytic inflammatory cells into vascular wall, differentiation of these monocytic inflammatory cells into phagocytes and activation of the same, oxidative modulation and enzymatic modulation for lipid in blood vessel and evoking of local inflammation due to such modulation, progression of various inflammatory reaction, and excessive repair reaction or abnormal repair reaction involved with proliferation of smooth muscle in the vascular wall. It is therefore thought that condition of arteriosclerosis may also be involved with the particular inflammation and tissue-repair reaction. It has then been advised conventionally that treatment and control of pathogenesis elements of arteriosclerosis like hypertension, diabetes, hyperlipidemia, obesity and smoking is important in order to delay the progression of arteriosclerosis.

### [DISCLOSURE OF THE INVENTION]

### [PROBLEMS TO BE SOLVED BY THE INVENTION]

In addition to control of the foregoing individual pathogenesis elements of arteriosclerosis, the art still needs a fundamental prophylactic and treatment methods for positively inhibiting the progression of arteriosclerosis.

### [MEANS TO SOLVE THE PROBLEMS]

The present inventors earnestly conducted study in view of the foregoing problems known in the art, and has finally realize the means to effectively inhibit the progression of arteriosclerosis.

Accordingly, disclosed herein is a polypeptide comprising the amino acid sequence set out in SEQ ID. NO: 1 as a part thereof and expression level of the polypeptide is increased in blood vessel of mammal which are recovered from ischemic condition by reperfusion of blood. Preferably, the polypeptide disclosed herein is consisting of the amino acid sequence set out in SEQ ID. NO:1.

Particularly, the present invention defines a reagent or medicine comprising a polypeptide consisting of the amino acid sequence set out in SEQ ID NO. 1 for use in the prevention or treatment of vascular disease.

### [EFFECTS OF THE INVENTION]

According to such elements of the present invention, pathogenic mechanism of ischemic disease will be clarified and a polypeptide which is useful for developing reagent and medicine that are preferable for preventing and treating arteriosclerosis will be also realized.

### [BRIEF DESCRIPTION OF THE DRAWINGS]

Figure 1 (a) illustrates open reading frame in the polypeptide according to the present invention. Figure 1 (b) denotes names of primers used to determine nucleotide sequences of the polypeptide according to the presnt invention and the nucleotide sequences so determined.
Figure 2 illustrates the alignment between the conventional collectin polypeptides and the polypeptide according to the present invention.
Figure 3 illustrates a result of genomic southern analysis.
Figure 4 illustrates distribution in tissues on the polypeptide.
Figure 5 illustrates conservation of the polypeptides among mammalian species.
Figure 6 is a phylogenetic tree of collectins.
Figure 7 illustratively shows structure of the plasmid pNOW1-hCL-P1.
Figure 8 illustrates polypeptide expression at mRNA level in the ischemia/reperfusion model.
Figure 9 illustrates enhancement of polypeptide expression in the hypoxic exposure/reoxygenation model.
Figure 10 illustrates uptake of the polypeptide into cells at their expression sites.

### [BEST MODE FOR CARRYING OUT THE INVENTION]

In general, the polypeptide according to the present invention is a protein belonged to collectin family. Then, the collectin is a generic name of proteins having calcium ion (Ca²⁺)-dependent carbohydrate recognition region (CRD) and collagen-like region, and the member of these proteins is conceived to involve in basic immunity systems against a wide spectrum of microorganisms such as bacteria and viruses.

Collectins that have been identified heretofore include mannan-binding protein (MBP), surfactant protein A (SP-A), surfactant protein D (SP-D), conglutinin and the like. In particular, involvement in nonspecific immune responses of the lectins such as conglutinin has been suggested that, for example, the lectins may play important roles in neutralizing and removing the various microorganisms in infants having insufficient maternal antibodies and undeveloped specific defense systems [Super et al., Lancet, Vol.2, 1236-1239, 1989]. Moreover, it was reported that the host becomes liable to be infected by a reduction of the MBP concentration in blood due to genetic mutation of the MBP gene [Sumiya et al., Lancet, Vol.337, 1569-1570, 1991]. WO 00/11161 identified a collectin which was expected to exhibit anti-bacterial or anti-viral activity in the human body. WO 01/59107 identified scavenger receptors having an SR structure and a collectin-like structure which could be utilized in the elucidation of mechanisms of development of a wide variety of diseases.

The present inventors previously found that the conglutinin and the mannan-binding protein can inhibit infection and hemagglutination activity of H1 and H3 Type Influenza A viruses [Wakamiya et al., Glycoconjugate J., Vol.8, 235, 1991; Wakamiya et al., Biochem. Biophys. Res. Comm., Vol.187, 1270-1278, 1992]. Thereafter, the present inventors isolated a cDNA clone encoding the conglutinin, and found that closer correlation may exist between the conglutinin gene and various surfactant protein D gene [Suzuki et al., Biochem. Biophys. Res. Comm., Vol.191, 335-342, 1993].

As stated above, the polypeptide disclosed herein comprises the amino acid sequence set out in SEQ ID. NO:1 as a part thereof, preferably, it consists of the amino acid sequence set out in SEQ ID. NO:1.

Then the polypeptide disclosed herein comprises the following six variants with the original physiological activity.

Polypeptide variant consisting of the amino acid sequence (SEQ ID. NO:2) prepared by further adding three amino acids to N-terminal of the amino acid sequence set out in SEQ ID. NO:1 is disclosed herein.

Polypeptide variant consisting of the amino acid sequence (SEQ ID. NO:3) prepared by further adding eighteen amino acids to N-terminal of the amino acid sequence set out in SEQ ID. NO:1 is disclosed herein.

Polypeptide variant consisting of the amino acid sequence (SEQ ID. NO:4) prepared by further adding 127 amino acids to N-terminal of the amino acid sequence set out in SEQ ID. NO:1 is disclosed herein.

Polypeptide variant consisting of the amino acid sequence (SEQ ID. NO:5) prepared by further adding 138 amino acids to N-terminal of the amino acid sequence set out in SEQ ID. NO:1 is disclosed herein.

Polypeptide variant consisting of the amino acid sequence (SEQ ID. NO:6) prepared by further adding 220 amino acids to N-terminal of the amino acid sequence set out in SEQ ID. NO:1 is disclosed herein.

Polypeptide variant consisting of the amino acid sequence (SEQ ID. NO:7) prepared by further adding 228 amino acids to N-terminal of the amino acid sequence set out in SEQ ID. NO:1 is disclosed herein.

As noted hereinafter, since the polypeptide disclosed herein is from human body, in particular, human placenta tissues, that can be expected to offer anti-bacterial activity and viral activity in human body and can also be expected to use it as physiologically active pharmaceutical substance.

### [EXAMPLES]

Although the present invention will be described along with Examples thereof, as a matter of coure, the present invention will not be restricted anyway based solely on the disclosure of these Examples.

### Example 1: Screening of Human Placenta cDNA Library

First of all, nucleotide sequences of insert DNA in fetus heart clone (I.M.A.G.E. Consortium Clone ID 34472 ; Registration Number in GenBank/EST Data Base, R74387) was sequenced by employing M13 Universal Promer (Pharmacia, SEQ ID NO:8, 5'-fluorescein cgacgttgtaaaacgacggccagt-3') and M13 Reverse Primer (Pharmacia, SEQ ID NO:9, 5'-fluorescein caggaaacagctat gac-3').

Probes for digoxigenin (DIG)-labeled cDNA probe [Reverse Primer, acaatctgatgagaaggtgatg (SEQ ID NO: 10) and Forward Primer, acgaggggctggatgggacat (SEQ ID NO:11) were prepared with 392A DNA/RNA Synthesizer (Applied Biosystems). DIGs were labeled with PCR DIG Probe synthesis Kit (Boeringer Mannheim). Components employed for the reaction were indicated in the following Table 1.

**[TABLE 1]**

| | |
|---|---|
| Plasmid DNA (Clone W72977, 50ng/µl) | 2µl (100ng) |
| 10 × Buffer | 5µl |
| 25mM MgCl₂ | 5µl |
| dNTP (PCR Labeling Mix) | 5µl |
| 20µM Reverse Primer | 2.5µl |
| 20µM Forward Primer | 5µl |
| Water | 28µl |
| Taq Polymerase | 0.5µl |

PCR reaction performed 35 cycles with Zymoreactor (ATTO Corp.) wherein the single cycle consists of: 1 minute at 92°C, 1 minute at 55°C, and 2 minutes at 72°C.

Then, phage cDNA library derived from human placenta was titrated as follows. *Escherichia coli* Y1090r, 0.2 ml, which had been cultured at 37°C for 16 hours in mLB medium (LB medium (1 g tryptone, 0.5g yeast extract and 0.5 g NaCl in total volume of 100 ml) containing 10 mM MgSO₄ and 0.2% maltose) and 0.1 ml of cDNA library serially diluted with SM buffer (5.8 g NaCl, 2 g MgSO₄·7H₂O, 2 M Tris-HCl (pH 7.5) 25 ml, and 2% gelatin 5 ml in total volume of 1L) were incubated at 37°C for 15 minutes, then the mixtures were added to 2.5 ml of LB-TOP agarose (0.75% agarose/LB medium) to make homogenous solutions, and plated onto 90 mmϕLB Medium Plates (Iwaki Glass), (1.5% agar/LB medium). The added solutions were hardened at room temperature for 15 minutes, then incubated for 5 hours at 42°C. The plaques on each of the plates were counted, and the titer of the phage was calculated. Consequently, the titer calculated to be 2.1 x 10¹⁰ pfu/ml.

The screening was performed as follows using the foregoing probes.

First of all, *Escherichia coli* Y1090r⁻, 0.6 ml, which had been cultured at 37°C for 16 hours in mLB medium, and cDNA library diluted with SM buffer to 1 x 10⁵ pfu were incubated at 37°C for 15 minutes. Then the mixture was added to 7.5 ml of LB-TOP agarose (0.75% agarose) to make a homogenous solution. The solution was plated onto ten LB square plates of 140cm² (Nissui Seiyaku), hardened at room temperature for 15 minutes, then the plates were incubated for 5 hours at 42°C. After plaque formation of each of the plates was confirmed, the transfer to the nylon membranes was performed. The transfer was performed using Nytran 13N (Schleicher and Schuell Co.). The filters of 12.5cm x 9.0 cm in size were immersed in distilled water for 10 minutes to be wet, then the excess water was removed on Whatmann 3MM Paper, and the filters were placed on the plates having the plaques formed thereon. After standing for two minutes, the filters were removed and air-dried for 10 minutes. The phage DNA on the filters was denatured for 2 minutes with 0.2 M NaOH/1.5 M NaCl, followed by neutralization with 0.4 M Tris-HCl (pH 7.6) / 2 x SSC for 2 minutes and washing with 2 x SSC for 2 minutes. Thereafter, the phage DNA was fixed on the membrane by UV irradiation with GS GENE LINKER (BioRad).

Hybridization and detection of the signals were conducted as follows.

The filters were soaked in 2 x SSC, and the excess moisture was removed using Whatmann 3MM Paper. Then, the filters were placed in a hybridization bag and prehybridization at 68°C for one hour in a hybridization solution (5 x SSC, 1% blocking agent, 0.1% N-lauroyl sarcosine and 0.02% SDS) was performed. Subsequently, the hybridization solution was removed from the bag, and the hybridization solution containing DIG labeled cDNA probe at a concentration of 10 ng/ml was added thereto, and hybridization was proceeded at 55°C for 16 hours. After the hybridization was completed, the filters were washed in a solution of 2 x SSC/0.1% SDS for 5 minutes, twice; and further washed in a solution of 0.5 x SSC/0.1% SDS for 15 minutes, twice. Then, SDS was removed using DIG buffer I (100 mM Tris-HCl, 150 mM NaCl (pH 7.5)) for 1 minute, and the filters were blocked with DIG buffer II (1% blocking agent, DIG buffer I) for 30 minutes. After washing the filters with DIG buffer I for one minute, a solution of alkaline phosphatase labeled anti-DIG antibody (Boehringer Mannheim) which was diluted to 5000-fold in DIG buffer II was added, and the reaction between antigen and antibody were allowed for 30 minutes at room temperature. The filters were then washed twice with DIG buffer I for 15 minutes at room temperature. Through the subsequent treatment of the filters with DIG buffer III (100 mM Tris-HCl, 100 mM NaCl (pH 9.5), 50 mM MgCl₂) for 3 minutes, the concentration of magnesium ion (Mg²⁺) was elevated. A solution of NBT/BCIP (WAKO Chem., Co.) in DIG buffer III was added for color development, accordingly, 10 positive clones were identified. The plaques corresponding to these clones were excised from the plates and placed in the tubes containing 1 ml of SM buffer. After stirring for 10 minutes, each of the buffer solution was serially diluted with SM buffer, and 0.1 ml of the diluted solution was mixed with 0.2 ml cultures of *Escherichia coli* Y1090r⁻ which had been cultured in mLB medium for 16 hours at 37°C. The mixture was incubated for 15 minutes at 37°C, and then added to 2.5 ml of LB-TOP agarose to make a homogenous solution. The solution was plated into ten 90mmϕLB plates, hardened at room temperature for 15 minutes, then the plates were incubated for 5 hours at 42°C. Several plaques were obtained, and the secondary screening was performed essentially in accordance with the procedures of the primary screening.

### Example 2 : Sequencing of Nucleotide Sequence of Selected Clones

The plaque of the clone that was expected as being appropriate among the positive clones obtained in the above secondary screening was excised from the plates. Then it was placed into a tube containing distilled water 200 µl followed by stirring for 30 minutes at room temperature, then the tube was centrifuged at 15, 000 rpm for 5 minutes, and the supernatant was obtained therefrom.

The insert DNA was amplified by PCR with TaKaRa LA PCR Kit Ver.2 (TAKARA Syuzo, Co.) using the resulting supernatant as a template. Components (diluted with water to be final volume of 50 µl) employed for PCR are indicated in the following Table 2.

**[TABLE 2]**

| | |
|---|---|
| Supernatant | 27 µl |
| 10 x LA PCR Buffer II (Mg²⁺ free) | 5 µl |
| 25 mM MgCl₂ | 5 µl |
| dNTP Mix | 8 µl |
| 20 µM λgt11 Reverse Primer (SEQ ID NO:) | 2.5 µl |
| 20 µM λgt11 Forward Primer (SEQ ID NO:) | 2.5 µl |
| LA Taq polymerase | 0.5 µl |
| Water | Balance |

PCR reaction was performed using Gene Amp PCR System 9600 (Applied Biosystems), with 30 cycles of: 20 seconds at 98°C, and 5 minutes at 68°C. The PCR product was verified by the electrophoresis on 1% agarose gel, and purified through excising from the gel. For this purification step, Sephaglas BandPrep Kit (Pharmacia) was used.

The excised DNA fragment was incorporated into pCR2.1 vector of TA Cloning Kit (Invitrogen). The recombinant vector was transformed into TOP10F' cell included in the TA Cloning Kit (Invitrogen). The transformants were cultured in LB medium (containing 100 µg/ml ampicillin), and three kinds of plasmids were extracted by alkaline SDS method.

Thus obtained DNA was cleaved with restriction enzymes that were expected to be adequate, and each DNA fragment was incorporated into pUC18 vector followed by transformation into XL1-Blue cell. The transformants were cultured on LB medium (containing 100 µg/ml ampicillin), and the plasmids were extracted by alkaline SDS method.
CL-P1-2-1 resulted in a plasmid containing EcoRI-Hind III fragment and Hind III-EcoRI fragment.
CL-P1-3-4 resulted in a plasmid containing EcoRI-BamHI fragment, BamHI-SmaI fragment, SmaI-HindIII fragment, KpnI-Sau3AI fragment, Sau3AI-EcoRI fragment, EcoRI-KpnI fragment and EcoRI-SmaI fragment.
CL-P1-3-7 resulted in a plasmid containing EcoRI-BamHI fragment, BamHI-SmaI fragment, SmaI-HindIII fragment, KpnI-Sau3AI fragment, Sau3AI-EcoRI fragment, EcoRI-KpnI fragment and KpnI-EcoRI fragment.

The primers prepared were: M13 Universal Primer (SEQ ID NO: 8); M13 Reverse Primer (SEQ ID NO: 9) from the Autoread Sequencing Kit (Pharmacia); and the following FITC (Pharmacia, Fluore Prime) -labeled primers that were produced using a DNA/RNA synthesizer. Then the nucleotide sequences of the entire regions were sequenced with Autoread Sequencing Kit (Pharmacia) and Autosequencer (A.L.F.).
HPP 1: 5'-fluorescein-cgtgaaaatgaatggaagtgg-3' (SEQ ID NO: 12),
HPP 2: 5'-fluorescein-ttttatccattgctgttcctc-3' (SEQ ID NO: 13),
HPP 3: 5'-fluorescein-ctggcagtccccgaggtccag-3' (SEQ ID NO: 14),
HPP 5: 5'-fluorescein-gctggtccccccggagagcgt-3' (SEQ ID NO: 15)

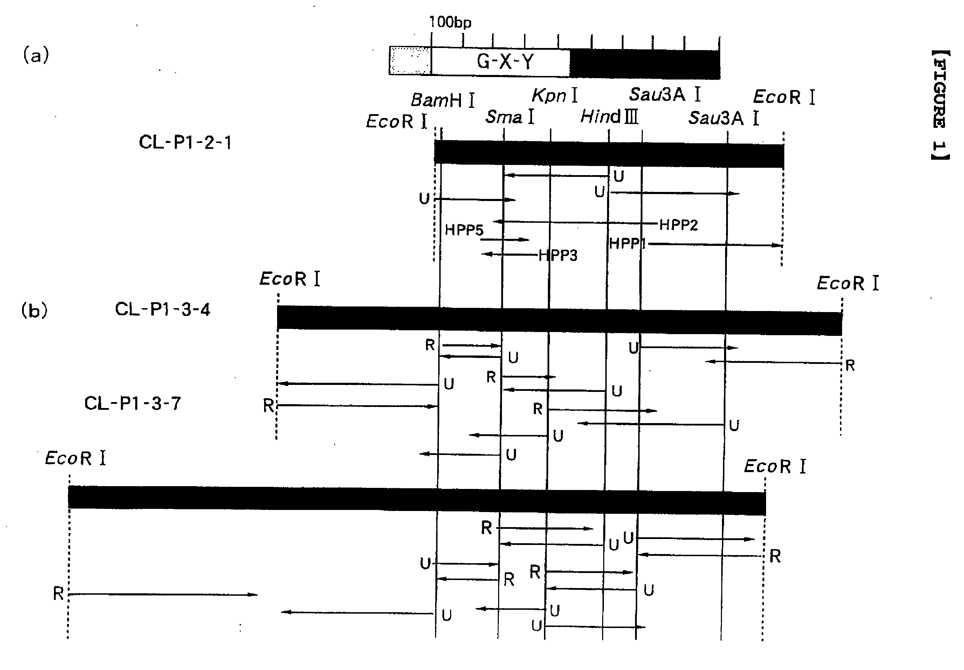

The outline of this nucleotide sequencing strategy performed is shown in Figure 1.

An open reading frame of the obtained collectin is illustrated in Figure 1 (a), wherein G-X-Y denotes a collagen-like region. Further, in Figure 1 (b), each of the primer names and positions of the nucleotide sequences that were read from the sequencer (shown as allows) are illustrated. M13 Universal Primer (shown as U) and M13 Reverse Primer (shown as R) are also illustrated.

In addition, a nucleotide sequence around the 5'-end region comprising a transcription initiation site was determined using Cap site cDNA.

First PCR was performed with Cap Site cDNA, 1RC2 Primer (5'-caaggtacgccacagcgtatg-3' (SEQ ID NO: 16)) attached to Human Liver (NIPPON GENE) and TGP1 Primer (5'-tcttcagtttccctaatccc-3' (SEQ ID NO: 17)) that was synthesized with Applied Biosystems 392A DNA/RNA Synthesizer.

Employed components (total volume of 50 µl) are indicated in the following Table 3.

**[TABLE 3]**

| |
|---|
| LA PCR Buffer II (Mg²⁺ free) |
| 2.5 mM MgCl₂ |
| each 200 µM of dATP, dCTP, dGTP and dTTP (TAKARA Syuzo, Co.), 1 µl |
| Cap Site cDNA Human Liver (NIPPON GENE) |
| 0.5 µM 1RC2 Primer (NIPPON GENE) |
| 0.5 µM TGP1 Primer |

PCR was performed using a program comprising 35 cycles of: heat denaturation for 20 seconds at 95°C, anealing for 20 seconds at 60°C, extension for 20 seconds at 72°C, with heat denaturation for 5 minutes at 95°C prior to the repeated reaction and final extension for 10 minutes at 72°C. After completing the first PCR, nested PCR was conducted.

The reaction was performed using 1 µl of the first PCR product as a template, with primers 2RC2 Primer (5'-gtacgccacagc gtatgatgc-3' (SEQ ID NO: 18)) attached and synthetic TGP2 Primer (5'-cattcttgacaaacttcatag-3' (SEQ ID NO: 19)) that was synthesized similarly to TGP1 Primer, and with the same reaction components and program (except that the cycle number was 25) as in the first PCR.

PCR reaction was performed with TaKaRa PCR Thermal Cycler 480 (TAKARA Syuzo, Co.). After thus obtained PCR product was confirmed on agarose gel electrophoresis, the band was excised from the gel, followed by freezing at -80°C for 10 minutes, centrifuge at 15000 rpm, for 10 minutes, and the supernatant was purified by ethanol precipitation.

The purified DNA fragment was incorporated into Novagen pT7Blue Vector, and the vector was transformed into competent XL1-Blue cell. The transformants were cultured on LB medium (containing 100 µg/ml ampicillin), and the plasmids were extracted by alkaline SDS method, followed by sequencing of the nucleotide sequence with Autoread Sequencing Kit (Pharmacia) and DNA Sequencer (A.L.F.). The employed primers were M13 Universal Primer (SEQ ID NO: 8) and M13 Reverse Primer (SEQ ID NO: 9) attached to AutoRead Sequencing Kit.

As a result, it was confirmed that the cDNA clone of the polypeptide that was obtained in Example 1 contained 2024 nucleotides set out in SEQ ID NO.20 which has open reading frame of 1026 nucleotides. It was also confirmed that a part of this nucleotide sequence comprises a region (739^{th}-1695^{th} nucleotides in the nucleotide sequence set out in SEQ ID NO.20) encoding 319 amino acids set out in SEQ ID NO: 1.

Results of homology searches of the DNA sequence and amino acid sequence on GenBank database revealed that the amino acid sequence of the obtained polypeptide is distinct from those of the collectins identified previously and it derives from a novel protein.

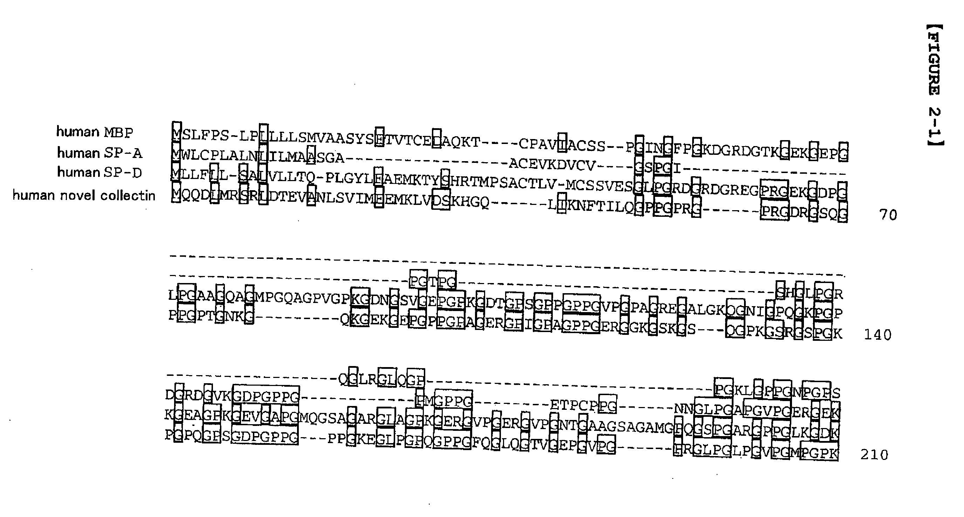

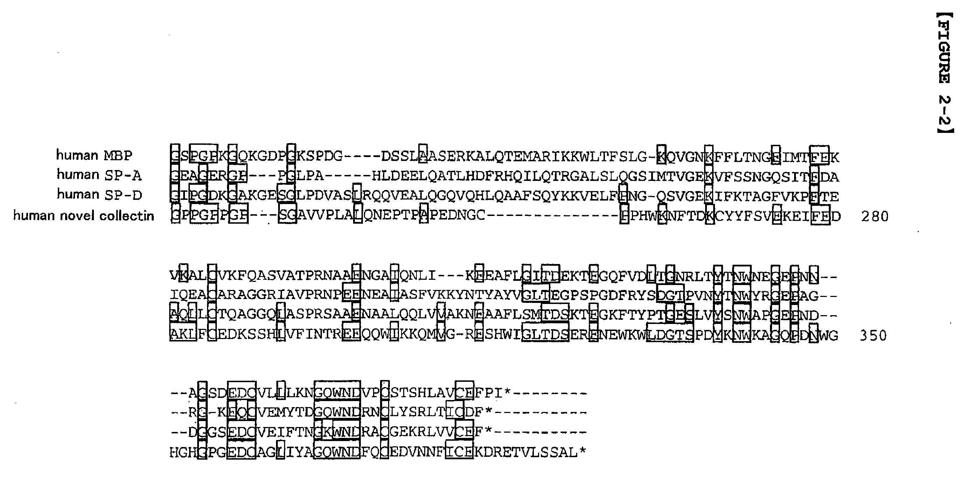

In addition, the amino acid sequence of the disclosed polypeptide was compared to those of three collectin proteins reported heretofore. The alignment is shown in Figure 2. Homologous amino acid residues were boxed. This alignment suggests that the obtained polypeptide shares homology with known collectins and it belongs to the collectin family.

### Vectors Available In The Present Invention

In addition to the vectors illustrated in the present Examples, any vector can be available as a matter of course provided that as far as it has been incorporated thereinto any polynucleotide encoding expression of the polypeptide disclosed herein or any active fragment thereof in order to allow expression of the polypeptide disclosed herein.

In other words, any vector in which the polynucleotide is inserted may be referred to as a replicon such as plasmid, λ-phage, cosmid and the like with the above described polynucleotide being incorporated therein so that the polynucleotide according to the present invention can be replicated and expressed. For example, vectors capable of cloning longer DNA fragments than cosmid may include P1 phage, F factor and artificial chromosome YAC of yeast and the like. Moreover, λ-phage may include replacement vector and insertion vector, and these can be selected ad libitum taking into account of the length of gene to be introduced.

Moreover, known vectors capable of being expressed in animal cells include for example, SV40 vectors, bovine papiloma virus vector, herpes virus vector, adenovirus vector, poxvirus vector, retrovirus vector and the like. On the other hand, commercially available vectors may include pUC19, pTV118N (Takara Shuzo Co., Ltd.), pUEX2 (Amersham), pGEX-4T, pKK233-2 (Pharmacia), pMAM-neo (Clontech), pGL2 (Promega), pDNA3.1+ (Invitrogen) and the like. A method of constructing such vectors can be performed utilizing restriction enzyme, ligase and the like by the conventional method without any particular limitation.

In the case where bacteria, in particular *Escherichia coli,* are used as a host cell that is transformed or transfected with the above described vector, the vector may usually comprise at least a promoter region (containing promoter, operator and Shine-Dalgarno sequence), initiation codon, a nucleotide sequence encoding the novel collectin, termination codon, terminator region and the like. In the case where yeast or an animal cell is used as a host, it is preferable that the vector includes at least promoter, initiation codon, nucleotide sequences encoding a signal peptide and the polypeptide according to the present invention, and termination codon. Moreover, enhancer sequence, 5'- and 3'-untranslated region of the polypeptide according to the present invention, splicing junction, polyadenylation site and selectable marker can be also inserted into the vector.

As a promoter incorporated into a vector of the present invention, conventionally well known SV40 (Simian Virus 40), SRα, cytomegalovirus (CMV) promoter, actin promoter, viral LTR (Long Terminal Repeat), for example, HTLV-1 LTR, HIV-LTR, Rous sarcoma virus LTR, herpes simplex tyrosine kinase virus promoter and the like can be used.

In the case where the expression is expected in eukaryotic cells of many cell types ranging from normal cells such as fibroblast, nerve cell, blood cell, parenchymal cell and the like to cancer cells, generally employed promoter may include cytomegalovirus, thymidine kinase (TK), β-actin and SV40 early gene promoter and the like. Since an enhancer is usually combined with a promoter sequence, it can be utilized as it is.

Moreover, in the case where cells and tissues in which the gene is introduced and expressed have been specified, a promoter specific for the cell can be selected. Furthermore, by combining these promoters in a homologous or heterologous manner, high expression can be expected, and an effect may be exerted that the polypeptide disclosed herein is stably obtained.

On the other hand, a promoter used in prokaryotic cells, PBAD, PL, trc, T7, SP6, T3, lac and the like may be included. In principle, a promoter that can result in high expression of the polypeptide disclosed herein in the cells from prokaryotic cells can be appropriately selected and used as a promoter in the present invention, as long as the promoter is compatible with the host. Moreover, in the case where cells and tissues in which the gene is introduced and expressed have been specified, a promoter specific for the cell can be selected.

Furthermore, by combining these promoters in a homologous or heterologous manner, high expression can be expected, and an effect may be exerted that the novel collectin is stably obtained.

On the other hand, promoter used in yeast may include GAL1, AOX1, CUP1, PGK and the like.

A selectable marker incorporated in the above described vector for recovering only the intended vectors may include dihydrofolate reductase (hereinafter abbreviated as DHFR) gene (methotrexate resistant gene), neo gene (G418 resistant gene) and the like, and for example, in the case where DHFR gene is used as a selectable marker employing CHO cell of which DHFR gene is deleted, the selection can be also performed by a thymidine free medium. In addition, when the gene is expressed in the cell by culturing in the medium at a higher methotrexate concentration and selecting resistant cells, the cell strain with higher expression can be also obtained.

### Example 3: Genomic Southern Analysis

Genomic Southern analysis was performed in order to clarify whether the gene encoding expression of the polypeptide taken in Example 2 was a single copy gene or a multi copy gene.

Four µg of genomic DNA (Promega) derived from human blood was digested with any one of the restriction enzymes, (1) EcoRI, (2) XbaI, (3) HindIII, (4) PstI, (5) BglII or (6) BamHI, followed by electrophoresis on 0.8% agarose gel at 100 mA, for 3 hours. After the electrophoresis was completed, they were transferred to a nylon membrane (Nytran 13N) to prepare a membrane for the analysis. For the transfer step, the electrophoresed gel was first immersed in 100 ml of 0.25 N HCl for 10 minutes, washed three times with distilled water, then immersed twice in 100 ml of a denaturalizing solution (1.5 M NaCl, 0.5 M NaOH) for 15 minutes, and immersed in 100 ml of a neutralizing solution (0.5 M Tris-HCl, 3 M NaCl (pH 6.8)) for 30 minutes so that depurination, denaturation and neutralization were accomplished. Thereafter, they were transferred using Vacuum Blotting System (Toyobo Engineering, VB-30). In this step, the membrane which had been pretreated by immersing in 2 x SSC for 5 minutes and in 20 x SSC for 5 minutes was used, with a pad which had been soaked with 20 x SSC. After the transfer was terminated, fixation of them was performed by UV irradiation.

Hybridization probe employed for the Southern analysis was the DNA probe [gaagacaagt cttcacatct tgttttcata aacactagag aggaacagca atggataaaa aaacagatgg tagggagaga gagccactgg atcggcctca cagactcaga g (SEQ ID NO: 23)] prepared by labeling primers [5'-gaagacaagtcttcaactcttg-3' (SEQ ID NO: 21) and 5'-ctctgagtctgtgaggccgatc-3' (SEQ ID NO: 22)] into a portion of open reading frame obtained in Example 2 with the above-mentioned PCR DIG Probe Synthesis Kit. Prior to hybridization, the probe was boiled for 10 minutes, and rapidly frozen with dry ice/ethanol for 5 minutes.

First, the membrane that was subjected to the transfer step was immersed in 2 x SSC for 5 minutes, then prehybridization was performed in ExpressHyb Hybridization Solution (Clonetech), 10 ml at 65°C for 30 minutes. Subsequently, the above frozen probe was diluted to 10 ng/ml in ExpressHyb Hybridization Solution, and 2 ml of this solution was used for hybridization at 65°C for one hour.

Following hybridization, the membrane was washed by: shaking in 20 ml of 2 x SSC, 0.1% SDS solution at room temperature for 5 minutes twice, then in 20 ml of 0.2 x SSC, 0.1% SDS solution at 65°C for 15 minutes twice. Next, the membrane was washed twice with 50 ml of DIG buffer I (100 mM Tris-HCl, 150 mM NaCl (pH 7.5)) at room temperature for one minute in order to remove SDS, and then blocked in 50 ml of DIG buffer II' (1.5% blocking agent in DIG buffer I) at room temperature for one hour.

Thereafter, the membrane was treated with 10 ml of alkaline phosphatase labeled anti-DIG antibody which had been diluted to 5000-fold in DIG buffer I containing 0.2% Tween20 for 30 minutes followed by washing twice by shaking in 50 ml of DIG buffer I which contains 0.2% Tween20 at room temperature for 20 minutes. After soaking the membrane twice in 10 ml of DIG buffer III at room temperature for 3 minutes, it was placed in a hybridization bag, and CSPD (registered trade mark, Boehringer Mannheim: chemiluminescence substrate) that had been diluted to 100-fold in DIG buffer III was added thereto so that the solution can spread over the membrane. Subsequently, the membrane was exposed onto Instant Film T612 (Polaroid).

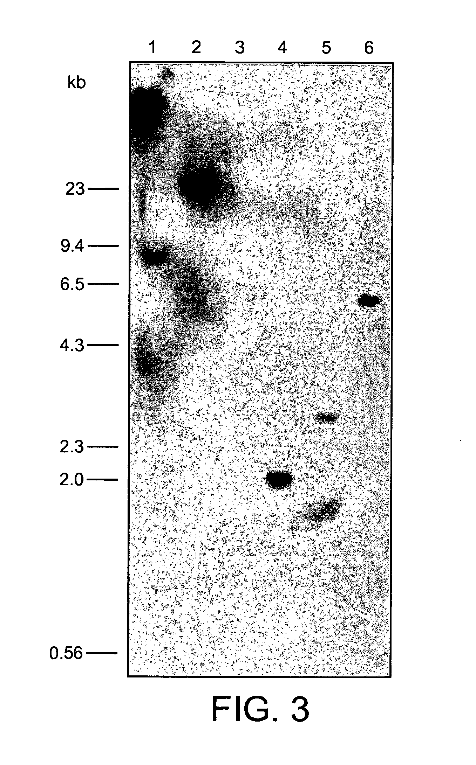

Consequently, it was speculated that the gene of the obtained polypeptide disclosed herein has been a single copy gene, because only one or two signals could be detected from the respective genomic DNA which was digested with each of the restriction enzymes, as shown in the lanes of Figure 3.

### Example 4 : Analysis of Expression Distribution in Human Tissues

In order to examine the expression of the mRNA of the polypeptide disclosed herein in the human tissues, analysis was performed by RT-PCR.

First of all, the various human tissues (organs) listed in the following 4 was prepared. In the meantime, tissue of the colon in lane 10 was purchased from OriGene Technologies, Inc. RT-PCR was performed using RNA LA PCR Kit (AMV) Ver.1.1 (TAKARA Syuzo, Co.) with RNA respectively derived from these human tissues as a template.

**[TABLE 4]**

| Lane Number (Figure 4) | Source Organ or Tissue |
|---|---|
| 1 | brain |
| 2 | heart |
| 3 | kidney |
| 4 | spleen |
| 5 | liver |
| 6 | small intestine |
| 7 | muscle |
| 8 | testis |
| 9 | placenta |
| 10 | colon |

First, reverse transcription reaction was conducted in the following reaction mixture (total volume of 20 µl was finally made with distilled water) noted in the following Table 5.

**[TABLE 5]**

| |
|---|
| 5 mM MgCl₂ |
| 1 x RNA PCR Buffer |
| 1 mM dNTP Mixture |
| 1 U/µl Rnase inhibitor |
| 0.25 U/µl reverse transcriptase |
| 0.125 µM Oligo dT-Adaptor Primer |
| RNA 1 µg |
| RNase free distilled water |

At the same time, a reaction mixture without reverse transcriptase was also prepared for the negative control.

The reaction mixture was placed in 0.2 ml tube, and subjected to PCR with TaKaRa PCR Thermal Cycler PERSONAL (TAKARA Syuzo, Co.) through 1 cycle of: 30 minutes at 42°C, 5 minutes at 99°C, and 5 minutes at 5°C. Thus resulted PCR product was subsequently used for LA PCR with the reaction mixture noted in the following Table 6.

**[TABLE 6]**

| |
|---|
| 2.5 mM MgCl₂ |
| 1 x RNA PCR Buffer II (Mg²⁺ free) |
| 2U TaKaRa LA Taq |

Two kinds of 0.2 µM primers (RT-PCR Primer U: 5'-gtgcccctggccctgcagaatg-3' (SEQ ID NO: 24) and RT-PCR Primer R: 5'-gcatatcaccctggggaacattttag-3' (SEQ ID NO: 25)) that was likely to amplify a cDNA sequence spanning from neck region to carbohydrate recognition domain of cDNA sequence of the polypeptide so produced has been added. Then they were adjusted to total volume of 80 µl with sterile distilled water.

PCR has initiated with one cycle of 2 mintes at 94°C and subsequent 50 cycles of: 30 seconds at 94°C, 30 seconds at 60°C, and 1 minute and 30 seconds at 72°C. The reaction product was separated on 1% agarose gel electrophoresis, followed by staining with ethidium bromide solution (0.1 µg/ml), verification of the electrophoretic pattern with transilluminator, and identification of the expressed tissues.

Further, in order to compare the expressed amount in each of the tissues, RT-PCR was performed to amplify a part of β-actin with each of the tissues, and the amount of RNA was corrected. This method was performed similarly to the above procedure with reverse transcriptase reaction, PCR reaction, and identified using 1% agarose gel electrophoresis as described above. Reaction mixture (total volume of 60 µl was finally made with distilled water) for reverse transcription reaction was noted in the following Table 7.

**[TABLE 7]**

| |
|---|
| 5 mM MgCl₂ |
| 1 x RNA PCR Buffer |
| 1 mM dNTP Mixture |
| 1 U/µl Rnase inhibitor |
| 0.25 U/µl reverse transcriptase |
| 2.5 µM Random 9mer |
| RNA 10ng |
| RNase free distilled water |

PCR was performed through one cycle of 10 minutes at 30°C, 15 minutes at 42°C, 5 minutes at 99°C, and 5 minutes at 5°C.

PCR reaction has then been conducted with PCR products so obtained with the reaction mixture (total volume of 40 µl was finally made with distilled water) noted in the following Table 8.

**[TABLE 8]**

| |
|---|
| 2.5 mM MgCl₂ |
| 1 x LA PCR Buffer (Mg²⁺ free) |
| 2U TaKaRa LA Taq |
| 0.25 µM human β-actin sense primer (SEQ ID NO: 24) |
| 0.25 µM human β-actin antisense primer (SEQ ID NO: 25) |
| distilled wate |

PCR was performed through 30 cycles of: 15 seconds at 94°C, and 30 seconds at 68°C.

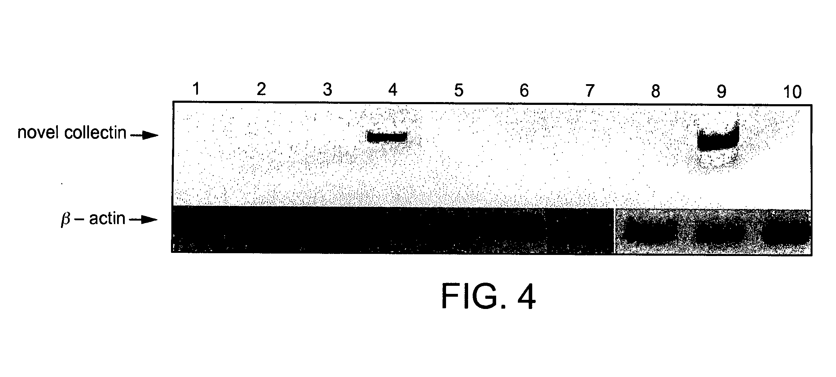

The results are shown in Figure 4, suggesting that mRNA of the polypeptide disclosed herein has been expressed in placenta (lane 9), spleen (lane 4), and kidney (lane 3). Extremely high expression in placenta is clearly suggested.

### Example 5: Genomic Southern Analysis

In order to elucidate conservation of the gene encoding the polypeptide disclosed herein in the other species of animals, analysis by genomic Southern hybridization was performed.

Hybridization probe employed for this analysis was the DIG labeled DNA probe which was corresponding to the open reading frame as described above and was labeled using PCR DIG Probe Synthesis Kit (Boehringer Mannheim). The employed membrane was prepared by treating each 5 µg of genomic DNAs from the animals noted in the following Table 9 with restriction enzyme EcoRI, subjecting those to electrophoresis on agarose gel, transfer to Nytran 13N membrane, and fixation by UV irradiation.

**[TABLE 9]**

| Lane Number (Figure 5) | Source Animals (Agency) |
|---|---|
| 1 | human (Promega) |
| 2 | monkey (Clonetech) |
| 3 | rat (Promega) |
| 4 | mouse (Promega) |
| 5 | dog (Clonetech) |
| 6 | cow (Promega) |
| 7 | rabbit (Clonetech) |
| 8 | chicken (Promega) |

Using such probes and membranes, hybridization were performed.

First, the membrane was immersed in 2 x SSC for 5 minutes, then prehybridization was performed in 10 ml of ExpressHyb Hybridization Solution at 65°C for 30 minutes. Subsequently, the probe that had been frozen as described above was diluted in the ExpressHyb Hybridization Solution to be 10 ng/ml, and 2 ml of thus diluted probe solution was used for hybridization at 65°C for one hour.

Following hybridization, the membrane was washed by: shaking in 20 ml of 2 x SSC, 0.1% SDS at room temperature for 5 minutes twice, and then shaking in 20 ml of 0.2 x SSC, 0.1% SDS at 68°C for 15 minutes twice. Next, the membrane was washed twice with DIG buffer I at room temperature for one minute in order to remove SDS, and was blocked in 50 ml of DIG buffer II' at room temperature for one hour.

Thereafter, the membrane was treated with 10 ml of alkaline phosphatase labeled anti-DIG antibody which had been diluted to 5000-fold in DIG buffer I which contains 0.2% Tween20 for 30 minutes followed by washing twice with shaking in 50 ml of DIG buffer I which contains 0.2% Tween20 at room temperature for 20 minutes. After soaking the membrane twice in 10 ml of DIG buffer III at room temperature for 3 minutes, it was placed in a hybridization bag, and CSPD that was diluted to 100-fold in DIG buffer III was added thereto so that the solution can spread over the membrane. Subsequently, the membrane was exposed to Instant Film T612.

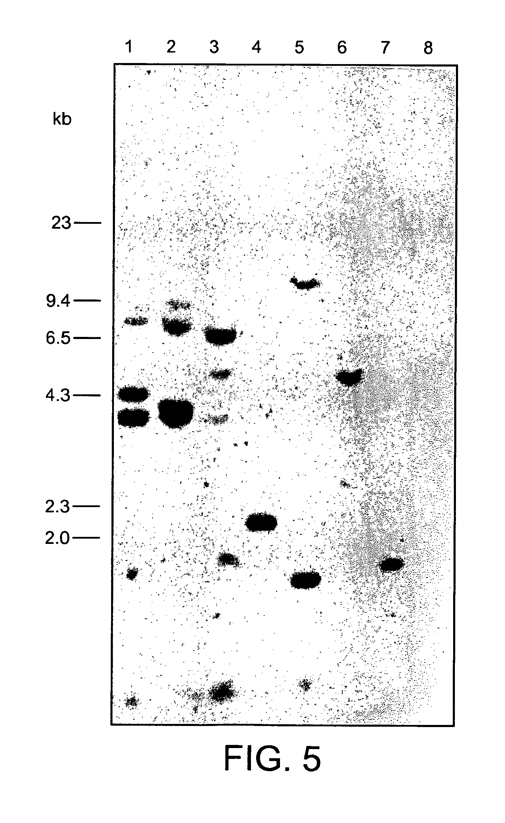

The result of this analysis is shown in Figure 5, wherein signals of DNA can been found in all species except for chicken (lane 8). Therefore, it was demonstrated that the polypeptide gene disclosed herein has been conserved between the mammalian species.

### Example 6: Genetic Analysis

To elucidate the genetic positional relevance of the present invention among the known collectins, analysis was performed based on DNA sequence of the gene encoding the polypeptide disclosed herein, and a phylogenetic tree was created.

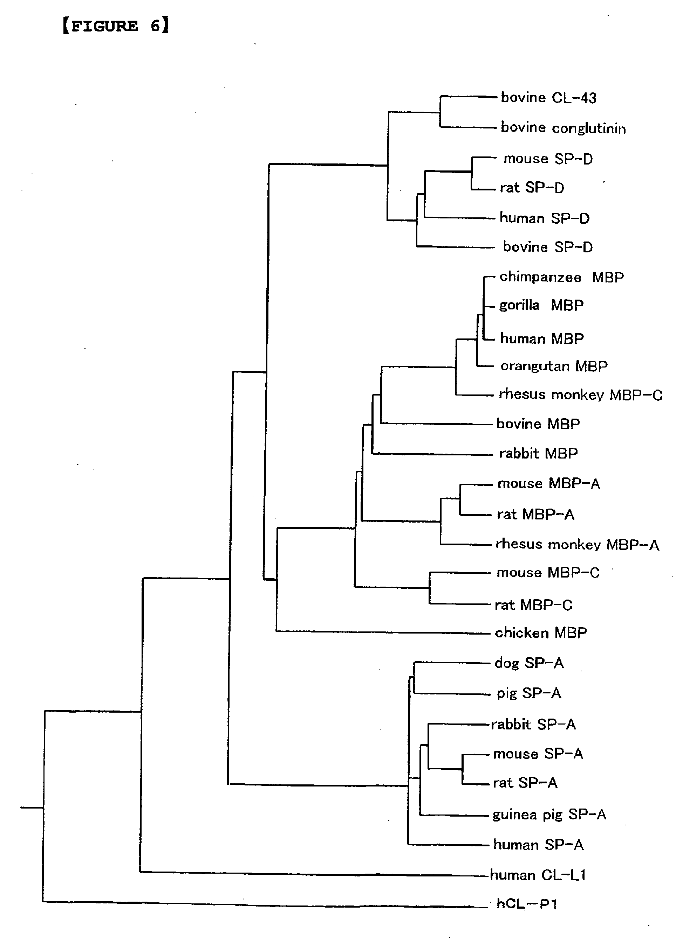

Collectins selected as subjects for analysis were several kinds of proteins belonging to the collectin family shown in Figure 6. In the meantime, in Figure 6, CL-L1 designates a collectin derived from human liver, which had been isolated by the present inventors (See, Japanese Patent Provisional Publication No. 11-206377).

Each of the amino acid sequence was retrieved from GenBank database, and then, multiple alignments were produced by clustalw method using the regions containing lectin domains from the obtained data. Then the phylogenetic tree was created with N-J (neighbor-joining) method and Phylip Version 3.57c package program.

Although SP-D, bovine collectin-43 and bovine conglutinin have constituted single cluster, and additional MBP and SP-A have respectively constituted separate clusters, any gene encoding the polypeptide disclosed herein has not belonged to any of these clusters similarly to CL-L1. Furthermore, it was speculated that the polypeptide disclosed herein may constitute a distinct cluster which is genetically diverse from those of the collectins reported heretofore including CL-L1.

### Example 7: Construction of Expression Vector

First, a sequence spanning from the initiation codon to termination codon of the gene set out in SEQ ID NO: 20 is amplified using a primer consisted of the nucleotide sequence: aaggaaaaaa gcggccgcat gcaacaagat ttgatgagg (SEQ ID NO: 26), and a primer consisted of the nucleotide sequence gctctagatt ataatgcaga tgacagtac (SEQ ID NO: 27) with Zymoreactor (ATTO Corp.).

The cDNA of the polypeptide so obtained is digested with restriction enzymes NotI and XbaI, yielding a corresponding portion in cDNA of the polypeptide disclosed herein (670^{th}-1698^{th} nucleotides in the nucleotide sequence set out in SEQ ID NO:20), which may be an insert.

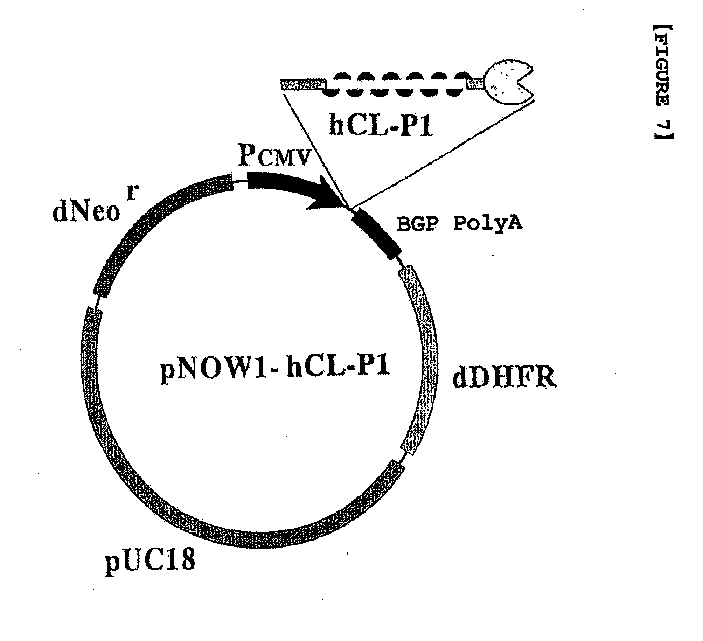

Expression vector pNOW/CMV-A disclosed in Japanese Patent Provisional Publication No. 10-179169 is then digested with restriction enzymes NotI and XbaI, and the above insert is inserted at downstream of cytomegalovirus promoter (pCMV), namely, between P_{CMV} (cytomegalovirus major antigen early promoter) and BGP poly A (bovine growth hormone polyadenylation signal), using a DNA ligation kit (Takara Shuzo Co., Ltd.). The expression vector thus obtained is designated as plasmid pNOW1-hCL-P1, and a schematic view of its structure is shown in Figure 7.

### Example 8: Selection of Producing Clone

### (1) Introduction of Expression Vector pNOW1-hCL-P1 Into Host Cells

Iscove' s Modified Dulbecco' s Medium (IMDM, GIBCO) with 10% fetal calf serum (FCS, GIBCO) and a medium (GIBCO) without hypoxanthine and thymidine are prepared, to which DHFR gene deficient DG44 Chinese Hamster Ovary (CHO) cell strains (dhfr⁻) are mixed to yield 1 x 10⁵ cells/ml, which is then seeded into a dish having 60 mm diameter, and is cultured at 37°C for 24 hours under a condition of 5% carbon dioxide (CO₂).

Culture medium supernatant is discarded, and in turn, IMDM containing 100 µl solution separately and previously obtained by mixing 5 µg of DNA (plasmid pNOW1-hCL-P1) with lipofectin solution (DOTAP Liposomal Transfection Reagent; Boeringer Mannheim) with 10% FCS is added to be 6 ml, further, hypoxanthine (final concentration of 10 nM) (GIBCO) and thymidine (final concentration of 100 nM) (GIBCO) are added thereto, followed by culturing for 16 hours, to introduce the plasmid pNOW1-hCL-P1 into the host dhfr⁻ CHO cells. Thereafter, the culture medium supernatant is discarded, followed by addition of 6 ml of IMDM added with 10% FCS, hypoxanthine, and thymidine, and further culture for 24 hours.

### (2) Collection of Nomycin (G418) Resistant CHO Cells

After the cells in which the expression vector pNOW1-hCL-P1 is incorporated are cultured for 24 hours, which are trypsinized and recovered from the dish, number of the cells are counted, and thereafter the mixture in which those cells are suspended in IMDM containing neomycin (G418) at a concentration of 400 µg/ml with 10% FCS is seeded (dispensed) into ten 96-well micro plates at an amount of 0.1 ml/well. The plates are cultured at 37C under a condition of 5% carbon dioxide (CO₂), and after two weeks, the surviving cells are collected as G418 resistant cells (clone).

These G418 resistant clones are identified for producibility of the polypeptide disclosed herein.

Several clones are selected among the clones in which the production of the polypeptide disclosed herein is confirmed, and then each of the selected clones is seeded into a culture flask of 25 cm². The culture is carried out until the cells become confluent (approximately 3 x 10⁶ cells/25 cm² culture flask). The culture medium supernatant from each culture flask is discarded, then 2 ml of the IMDM containing 10% FCS identical to the composition described above is added thereto, followed by culture for four days, and collecting the culture medium supernatant. The produced amount of the polypeptide disclosed herein (rhCL-P1: recombinant human polypeptide) in the collected culture medium supernatant is determined.

It should be noted that an amount of the produced polypeptide disclosed herein may be quantitatively determined according to the method of Suzuki *et al.* (Y. Suzuki, et al., "Characterization of Recombinant Bovine Conglutinin Expressed in a Mammalian Cell", Biochem. Biophys. Res. Commun., 238:856-863 (1997)). In particular, using an anti-rabbit polyclonal antibody to the polypeptide disclosed herein expressed in *Escherichia coli,* carbohydrate recognition domain (CRD) and neck region of collectins (expressed also in *Escherichia coli* using a similar method), as well as the polypeptide (the subject to be quantitated)disclosed herein, quantitation is performed along with the methodology noted in the same thesis.

### (3) Collection of Methotrexate Resistant CHO Cells

Following further passage culture and stabilization of the clones which produce the polypeptide disclosed herein and are obtained in the above-described Example 8 (2), low concentration of methotrexate (MTX) is added to the medium, and gene amplification is performed.

First, the selected each cell clone is mixed in IMDM containing 5 nM MTX, 400 µg/ml of G418 with 10% FCS previously dialyzed (JRH Bioscience), is seeded (dispensed) into ten 96-well micro plates at 0.1 ml/well. The cells are cultured at 37°C under a condition of 5% carbon dioxide (CO₂), and after two weeks, the surviving cells are collected as 5 nM MTX resistant cells (clones).

These 5 nM MTX resistant clones are identified for their producibility on the polypeptide disclosed herein.

Several clones are optionally selected among the clones in which the production of the polypeptide disclosed herein is confirmed, and then each of the selected clones is seeded into a culture flask of 25 cm² followed by two weeks culture until the cells become confluent. The culture medium supernatant is discarded, then 2 ml of the IMDM (5nM MTX and 400µg/ml G418 are added) containing 10% FCS identical to the composition described above is added thereto, followed by culture for four days and collection of the culture medium supernatant, and production level of the polypeptode disclosed herein is determined.

### (4) Determination of Quantity of Production of Polypeptide According to Present Invention

Emulsion are prepared by mixing 100µl of PBS (-) (PBS without calcium ion and magnesium ion) containing 100µg of the polypeptide disclosed herein disolved therein with 100µl of Complete Freund's adjuvant and the emulsion are injected into abdominal cavity of two BALB/c mice (female, eight weeks old).

Three weeks later, the second immunization is similarly performed. At that time, instead of Complete Freund' s adjuvant, Incomplete Freund's adjuvant is used.

Two weeks later thereafter, serum was collected, and antibody titer thereof is determined. Mouse having the highest antibody titer is selected, and the final immunization is similarly performed one week later. Five days later from the final immunization, spleen is removed and suspended cells are prepared. Cell fusion is performed by mixing 2×10⁷ cells of myeloma cell NS-1 cell with 2×10⁸ cells of splenic cell so prepared. Mixture is centrifuged at 1, 600rpm for 6 minutes at room temperature, supernatant is then removed, 1 ml of polyethyleneglycol (PEG) is added to the remined cells just soften for 1 minute, and it is further agitaed for 1 munute at room temperature. Then, 1ml of base medium (Iscove' s medium, 15% FCS, 2ml L-glutamin solution, 0.05mM 2-mercaptoethanol) which is kept previously at 37°C is dropped for 1 minute with slightly shaking it. After repeating this operation onece again, similar step is performed with 8 ml of the same for 3 minutes. PEG is then removed by centrifuging it at 1, 000rpm for 5 minutes at room temperature.

Pellet is then soften and suspension was prepared by adding thereto 5×10⁶ cells/ml of thymic cell suspension (solution prepared by suspending cells in hybridoma growth medium containing HAT and used as feeder cell to increase hybridoma efficiency). 0.1 ml of this suspension was seeded into each well of 96-well plate and was cultivated in a carbon dioxide (CO₂) incubator at 37°C under 5% carbon dioxide.

Selection of hybridoma is performed as follows.

At day after commencing cultivation, 150µl of HAT medium (a hybridoma growth medium containing HAT) is added to each well, and cultivation is further continued for two days. Two days later, the medium is substituted by removing 150µl of the medium and supplementing 150µl of HAT medium, and similar substitution on the medium is performed when cultivation is continued for further three days and for further seven days. Four days later thereafter, 100µl of supernatant is collected, and screening is performed for determining antibody titer to the polypeptide according to the present invention. Antibody positive cells are then transferred from the 96-well plate to 24-well multi plate with Pasteur pipette. At that time, 0.5 ml of HT medium (a hybridoma growth medium containing hypoxanthine and thymidine) is previously added to each well.

After cultivating them for three days, a part of the medium is collected, and cells which can be judged as antibody positive from reexamination on their antibody titer are subjected to cloning and freeze preservation. Cloning is performed by diluting positive cells with suspension of thymic cells through limiting dilution, then adjusting them into one cell/ml, and seeding 0.2 ml of those into each well of 96-well pate. After cultivating them for ten days, antibody titer is then determined on the clone which forms single colony per well and two clones that show the higher antibody titer are selected.

Cloning is performed again on these two clonies. Cloning method is similar to that according to the first cloning. Cultivation scale on antibody positive cells are then enlarged. They are transferred to 24-well plate, then are cultivated therein for three days, and are transferred to 25cm² flasks. Three days thereafter, they are transferred to 225cm² flasks and are cultivated therein for further three days. Monoclonal antibodies are then prepared by collecting supernatant.

Production of the monoclonal antibodies from ascites is performed by adjusting the cloned hybridoma into 1 × 10⁷ cells/ml and injecting interperitoneally them into mice at the amount of 1 ml/mouse. One week later, mice having the hypertrophied abdominal are anesthetized with ether and ascites is collected. Ascites is transferred to centrifuging tubes of procoagulant type containing serum separating agent, then is left for 30 minutes at room temperature, and are centrifuging at 3,000 rpm for 5 minutes. Antibody is obtained by collecting upper ascites and purifying those with ammonium sulfate fractionation.

Antibody (anti-mouse-anti-human polypeptide antibody) so produced is diluted into 10µg/ml with coating buffer (50mM carbonic acid-hydrogen carbonate buffer, pH 9.6), and 100µl of the antibody soltuion is added to each well of 96-well ELISA plate. Incubation is then performed overnight at 4°C. They are washed three times with 300µl of solution (TBS/T solution (25mM Tris-HCl, 0.14M NaCl, 5mM KCl, 0.05% Tween20, pH 7.4)). Following washing operations are similarly performed. 300µl of 5% skim milk/TBS/T solution is added thereto and is left for one hour. After washing them, 100µl of samples and standard polypeptide disclosed herein are respectively added thereto and they are left for one hour at 37°C. At this stage, the samples are appropriately diluted, while standard preparation is adjusted to 0.02µg/ml as the maximum concentration and two-fold serial dilution is employed. After washing them, 100µl of 1 µg/ml biotinylated anti-rabbit anti-human polypeptide antibody is added thereto and they are left for one hour at 37°C. After washing them, 100µl of biotin streptoavidin solution is added thereto and they are left for 30 minutes at 37°C. After further washing them, 100µl of 3, 3', 5, 5'-tetra-methyl-benzidine solution is added thereto and they are left for 30 minutes at room temperature. Thereafter, reaction is terminated by adding thereto 100µl of 1N phosphoric acid, and absorbance at 450nm wavelength is determined.

Calibration curve is prepared on the standard preparation according to this method, and an amount of the polypeptide disclosed herein so produced is determined.

### EXAMPLE 9: Relevancy of Polypeptides to Pathogenic Mechanism of Ischemic Disease

Various disorders such as activation of inflammatory cells like neutrophils and generation of active oxygen are topically appeared by reperfusing bloods after vascular ischemic disease like myocardial infarction is confirmed. Ischemia/reperfusion model employing rat or mouse has been produced as an animal model which realizes this circumstance. This model has often been used in study to clarify the mechanism of these disorders.

Then, after reperfusion of blood, an examination was performed with regard to dynamics of the polypeptide disclosed herein with ischemia/reperfusion model on carotid artery of rat.

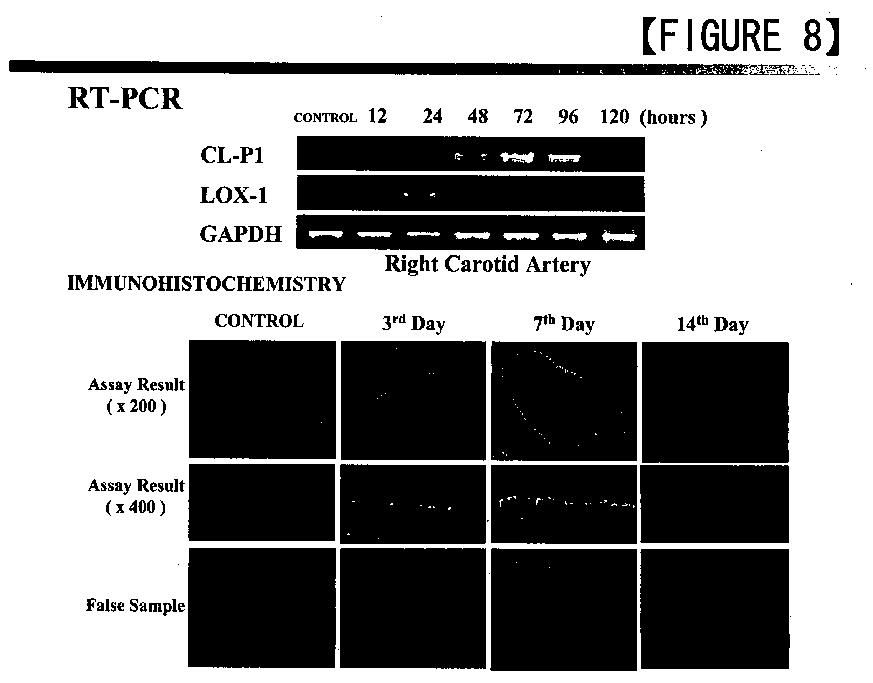

Firstly, ischemic condition was kept for 30 minutes on carotid artery of a rat (female, eight weeks old). Then, when blood has been reperfused, an examination was performed on time-coursely expression of mRNA involved in expression of the polypeptide disclosed herein as well as time-coursely expression on the protein from such mRNA. As the result thereof, as shown in Figure 8, expression of the polypeptide disclosed herein in carotid artery endothelium indicated that their expression level in mRNA level increased from 48 hours, reached optimum level at 72 hours, and returned into normal level at 120 hours. When the polypeptide disclosed herein expressed in vascular endothelial cells was examined with immunohistological staining, it was demonstrated that expression level increased at after three days and reached peak at seven days, and their expression character was still maintained at 14^{th} day or later. Any increase of expression has not been determined in the opposite carotid artery (control) of the same subject wherein ischemia/ reperfusion were not performed.

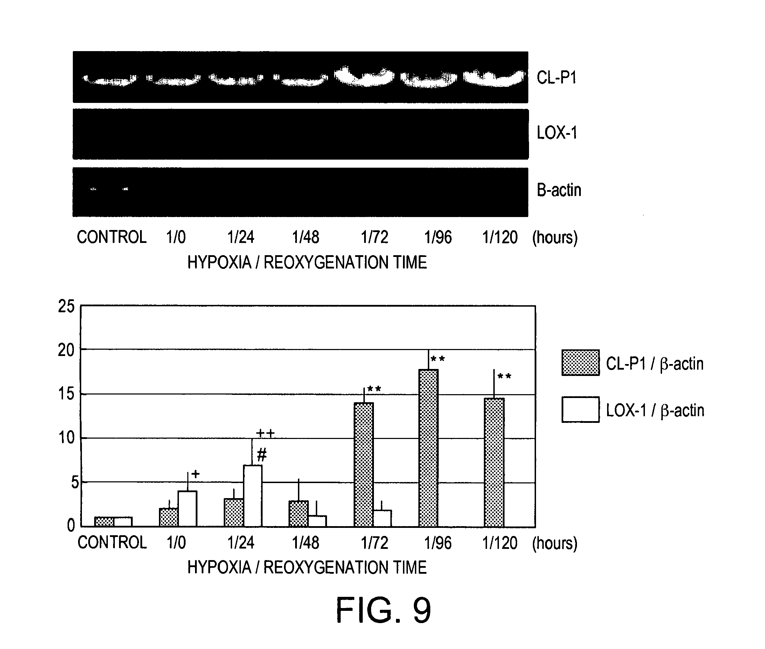

Similar results were obtained in hypoxic exposure/ reoxygenation model employing vascular endothelial cell HUVECs which is an *in vitro* ischemia/reperfusion model on carotid artery. Thus, obviously from the results shown in Figure 9, expression was increased even when hypoxic exposure was performed for 30 minutes and 72 hours was then past from reoxygenation.

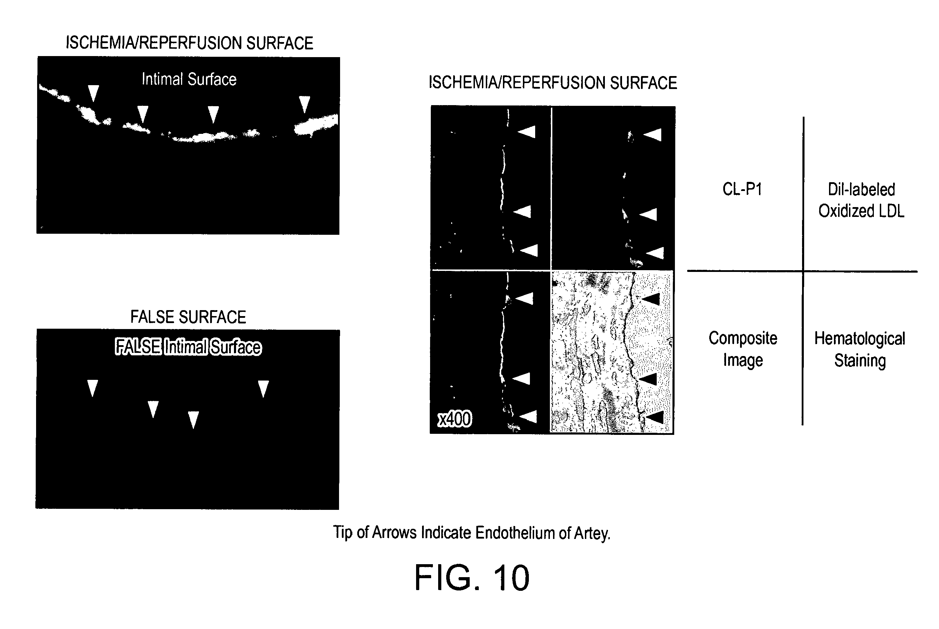

Effects of the polypeptide disclosed herein were suggested in view of the increased expression thereof through vasostimulation like reperfusion after ischemia. Accordingly, as demonstrated by the results shown in Figure 10, when a ligand like denatured low-density lipoprotein (denatured LDL) or oxidized low-density lipoprotein (oxidized LDL) was added at the expression was just increased and their dynamics were observed, it was found that such ligands reacts with expression sites of the polypeptide disclosed herein and they were taken into inside of cells.

From the foregoing disclosure, although the polypeptide disclosed herein will not offer effect at their usual expression level, their real effects will be produced by inducing topical expression with stimulation and increasing such expression. For example, the polypeptide disclosed herein may take oxidized LDL which induces arteriosclerosis and duly develop such arteriosclerosis. In such cases, administration of any polypeptide like that from binding domain which binds to extracellular domain of the polypeptide disclosed herein or any ligand thereof may limit such uptake and inhibit various vascular diseases including arteriosclerosis.

### [INDUSTRIAL APPLICABILITY]

Polypeptides disclosed herein contribute to resolution of pathogenic mechanism of ischemic diseases, in particular, diseases due to arteriosclerosis and can be employed to develop reagent and medicine which are suitable for prevention and treatment of these diseases.

## Claims

1. A reagent or medicine comprising a polypeptide consisting of the amino acid sequence set out in SEQ ID. NO. 1 for use in the prevention or treatment of vascular disease.

2. The reagent or medicine for use according to claim 1 wherein said disease is arteriosclerosis.

## Patentansprüche

1. Reagens oder Medikament umfassend ein Polypeptid, das aus der in SEQ ID NO: 1 aufgeführten Aminosäuresequenz besteht, zur Verwendung bei der Prävention oder Behandlung von Gefäßkrankheit.

2. Reagens oder Medikament nach Anspruch 1, wobei die Krankheit Arteriosklerose ist.

## Revendications

1. Réactif ou médicament comprenant un polypeptide consistant en la séquence d'acides aminés exposée dans SEQ ID n° 1 pour utilisation dans la prévention ou le traitement d'une maladie vasculaire.

2. Réactif ou médicament pour utilisation selon la revendication 1, dans lequel ladite maladie est l'artériosclérose.


### SEQUENCE LISTING

<110> FUSO PHARMACEUTICAL INDUSTRIES, LTD. WAKAMIYA, Nobutaka OTANI, Katsuki
<120> POLYPEPTIDE ACTING TO ENHANCE ISCHEMIC DISEASES
<130> 14.113934
<140> PCT/JP2011/001285
   <141> 2011-03-04
<150> JP 2010-047335
   <151> 2010-03-04
<160> 30
<210> 1
   <211> 319
   <212> PRT
   <213> Homo Sapiens
<400> 1
   Met Lys Leu Val Asp Ser Lys His Gly Gln Leu Ile Lys Asn Phe Thr
   1 5 10 15
   Ile Leu Gln Gly Pro Pro Gly Pro Arg Gly Pro Arg Gly Asp Arg Gly
   20 25 30
   Ser Gln Gly Pro Pro Gly Pro Thr Gly Asn Lys Gly Gln Lys Gly Glu
   35 40 45
   Lys Gly Glu Pro Gly Pro Pro Gly Pro Ala Gly Glu Arg Gly Pro Ile
   50 55 60
   Gly Pro Ala Gly Pro Pro Gly Glu Arg Gly Gly Lys Gly Ser Lys Gly
   65 70 75 80
   Ser Gln Gly Pro Lys Gly Ser Arg Gly Ser Pro Gly Lys Pro Gly Pro
   85 90 95
   Gln Gly Pro Ser Gly Asp Pro Gly Pro Pro Gly Pro Pro Gly Lys Glu
   100 105 110
   Gly Leu Pro Gly Pro Gln Gly Pro Pro Gly Phe Gln Gly Leu Gln Gly
   115 120 125
   Thr Val Gly Glu Pro Gly Val Pro Gly Pro Arg Gly Leu Pro Gly Leu
   130 135 140
   Pro Gly Val Pro Gly Met Pro Gly Pro Lys Gly Pro Pro Gly Pro Pro
   145 150 155 160
   Gly Pro Ser Gly Ala Val Val Pro Leu Ala Leu Gln Asn Glu Pro Thr
   165 170 175
   Pro Ala Pro Glu Asp Asn Gly Cys Pro Pro His Trp Lys Asn Phe Thr
   180 185 190
   Asp Lys Cys Tyr Tyr Phe Ser Val Glu Lys Glu Ile Phe Glu Asp Ala
   195 200 205
   Lys Leu Phe Cys Glu Asp Lys Ser Ser His Leu Val Phe Ile Asn Thr
   210 215 220
   Arg Glu Glu Gln Gln Trp Ile Lys Lys Gln Met Val Gly Arg Glu Ser
   225 230 235 240
   His Trp Ile Gly Leu Thr Asp Ser Glu Arg Glu Asn Glu Trp Lys Trp
   245 250 255
   Leu Asp Gly Thr Ser Pro Asp Tyr Lys Asn Trp Lys Ala Gly Gln Pro
   260 265 270
   Asp Asn Trp Gly His Gly His Gly Pro Gly Glu Asp Cys Ala Gly Leu
   275 280 285
   Ile Tyr Ala Gly Gln Trp Asn Asp Phe Gln Cys Glu Asp Val Asn Asn
   290 295 300
   Phe Ile Cys Glu Lys Asp Arg Glu Thr Val Leu Ser Ser Ala Leu
   305 310 315
<210> 2
   <211> 322
   <212> PRT
   <213> Homo Sapiens
<400> 2
   Met Glu Glu Met Lys Leu Val Asp Ser Lys His Gly Gln Leu Ile Lys
    1               5                  10                  15
   Asn Phe Thr Ile Leu Gln Gly Pro Pro Gly Pro Arg Gly Pro Arg Gly
               20                  25                  30
   Asp Arg Gly Ser Gln Gly Pro Pro Gly Pro Thr Gly Asn Lys Gly Gln
           35                  40                  45
   Lys Gly Glu Lys Gly Glu Pro Gly Pro Pro Gly Pro Ala Gly Glu Arg
       50                  55                  60
   Gly Pro Ile Gly Pro Ala Gly Pro Pro Gly Glu Arg Gly Gly Lys Gly
   65                  70                  75                  80
   Ser Lys Gly Ser Gln Gly Pro Lys Gly Ser Arg Gly Ser Pro Gly Lys
                   85                  90                  95
   Pro Gly Pro Gln Gly Pro Ser Gly Asp Pro Gly Pro Pro Gly Pro Pro
               100                 105                 110
   Gly Lys Glu Gly Leu Pro Gly Pro Gln Gly Pro Pro Gly Phe Gln Gly
           115                 120                 125
   Leu Gln Gly Thr Val Gly Glu Pro Gly Val Pro Gly Pro Arg Gly Leu
       130                 135                 140
   Pro Gly Leu Pro Gly Val Pro Gly Met Pro Gly Pro Lys Gly Pro Pro
   145                 150                 155                 160
   Gly Pro Pro Gly Pro Ser Gly Ala Val Val Pro Leu Ala Leu Gln Asn
                   165                 170                 175
   Glu Pro Thr Pro Ala Pro Glu Asp Asn Gly Cys Pro Pro His Trp Lys
               180                 185                 190
   Asn Phe Thr Asp Lys Cys Tyr Tyr Phe Ser Val Glu Lys Glu Ile Phe
           195                 200                 205
   Glu Asp Ala Lys Leu Phe Cys Glu Asp Lys Ser Ser His Leu Val Phe
       210                 215                 220
   Ile Asn Thr Arg Glu Glu Gln Gln Trp Ile Lys Lys Gln Met Val Gly
   225                 230                 235                 240
   Arg Glu Ser His Trp Ile Gly Leu Thr Asp Ser Glu Arg Glu Asn Glu
                   245                 250                 255
   Trp Lys Trp Leu Asp Gly Thr Ser Pro Asp Tyr Lys Asn Trp Lys Ala
               260                 265                 270
   Gly Gln Pro Asp Asn Trp Gly His Gly His Gly Pro Gly Glu Asp Cys
           275                 280                 285
   Ala Gly Leu Ile Tyr Ala Gly Gln Trp Asn Asp Phe Gln Cys Glu Asp
       290                 295                 300
   Val Asn Asn Phe Ile Cys Glu Lys Asp Arg Glu Thr Val Leu Ser Ser
   305                 310                 315                 320
   Ala Leu
<210> 3
   <211> 337
   <212> PRT
   <213> Homo Sapiens
<400> 3
   Met Arg Ser Arg Leu Asp Thr Glu Val Ala Asn Leu Ser Val Ile Met
    1               5                  10                  15
   Glu Glu Met Lys Leu Val Asp Ser Lys His Gly Gln Leu Ile Lys Asn
               20                  25                  30
   Phe Thr Ile Leu Gln Gly Pro Pro Gly Pro Arg Gly Pro Arg Gly Asp
           35                  40                  45
   Arg Gly Ser Gln Gly Pro Pro Gly Pro Thr Gly Asn Lys Gly Gln Lys
       50                  55                  60
   Gly Glu Lys Gly Glu Pro Gly Pro Pro Gly Pro Ala Gly Glu Arg Gly
   65                  70                  75                  80
   Pro Ile Gly Pro Ala Gly Pro Pro Gly Glu Arg Gly Gly Lys Gly Ser
                                   85                  90                  95
   Lys Gly Ser Gln Gly Pro Lys Gly Ser Arg Gly Ser Pro Gly Lys Pro
                   100                 105                 110
   Gly Pro Gln Gly Pro Ser Gly Asp Pro Gly Pro Pro Gly Pro Pro Gly
           115                 120                 125
   Lys Glu Gly Leu Pro Gly Pro Gln Gly Pro Pro Gly Phe Gln Gly Leu
       130                 135                 140
   Gln Gly Thr Val Gly Glu Pro Gly Val Pro Gly Pro Arg Gly Leu Pro
   145                 150                 155                 160
   Gly Leu Pro Gly Val Pro Gly Met Pro Gly Pro Lys Gly Pro Pro Gly
                   165                 170                 175
   Pro Pro Gly Pro Ser Gly Ala Val Val Pro Leu Ala Leu Gln Asn Glu
               180                 185                 190
   Pro Thr Pro Ala Pro Glu Asp Asn Gly Cys Pro Pro His Trp Lys Asn
           195                 200                 205
   Phe Thr Asp Lys Cys Tyr Tyr Phe Ser Val Glu Lys Glu Ile Phe Glu
       210                 215                 220
   Asp Ala Lys Leu Phe Cys Glu Asp Lys Ser Ser His Leu Val Phe Ile
   225                 230                 235                 240
   Asn Thr Arg Glu Glu Gln Gln Trp Ile Lys Lys Gln Met Val Gly Arg
                   245                 250                 255
   Glu Ser His Trp Ile Gly Leu Thr Asp Ser Glu Arg Glu Asn Glu Trp
               260                 265                 270
   Lys Trp Leu Asp Gly Thr Ser Pro Asp Tyr Lys Asn Trp Lys Ala Gly
           275                 280                 285
   Gln Pro Asp Asn Trp Gly His Gly His Gly Pro Gly Glu Asp Cys Ala
       290                 295                 300
   Gly Leu Ile Tyr Ala Gly Gln Trp Asn Asp Phe Gln Cys Glu Asp Val
   305                 310                 315                 320
   Asn Asn Phe Ile Cys Glu Lys Asp Arg Glu Thr Val Leu Ser Ser Ala
                   325                 330                 335
   Leu
<210> 4
   <211> 446
   <212> PRT
   <213> Homo Sapiens
<400> 4
   Met Glu Asn Ile Thr Thr Ile Ser Gln Ala Asn Glu Gln Asn Leu Lys
    1               5                  10                  15
   Asp Leu Gln Asp Leu His Lys Asp Ala Glu Asn Arg Thr Ala Ile Lys
               20                  25                  30
   Phe Asn Gln Leu Glu Glu Arg Phe Gln Leu Phe Glu Thr Asp Ile Val
           35                  40                  45
   Asn Ile Ile Ser Asn Ile Ser Tyr Thr Ala His His Leu Arg Thr Leu
       50                  55                  60
   Thr Ser Asn Leu Asn Glu Val Arg Thr Thr Cys Thr Asp Thr Leu Thr
   65                  70                  75                  80
   Lys His Thr Asp Asp Leu Thr Ser Leu Asn Asn Thr Leu Ala Asn Ile
                   85                  90                  95
   Arg Leu Asp Ser Val Ser Leu Arg Met Gln Gln Asp Leu Met Arg Ser
               100                 105                 110
   Arg Leu Asp Thr Glu Val Ala Asn Leu Ser Val Ile Met Glu Glu Met
           115                 120                 125
   Lys Leu Val Asp Ser Lys His Gly Gln Leu Ile Lys Asn Phe Thr Ile
       130                 135                 140
   Leu Gln Gly Pro Pro Gly Pro Arg Gly Pro Arg Gly Asp Arg Gly Ser
   145                 150                 155                 160
   Gln Gly Pro Pro Gly Pro Thr Gly Asn Lys Gly Gln Lys Gly Glu Lys
                   165                 170                 175
   Gly Glu Pro Gly Pro Pro Gly Pro Ala Gly Glu Arg Gly Pro Ile Gly
               180                 185                 190
   Pro Ala Gly Pro Pro Gly Glu Arg Gly Gly Lys Gly Ser Lys Gly Ser
           195                 200                 205
   Gln Gly Pro Lys Gly Ser Arg Gly Ser Pro Gly Lys Pro Gly Pro Gln
       210                 215                 220
   Gly Pro Ser Gly Asp Pro Gly Pro Pro Gly Pro Pro Gly Lys Glu Gly
   225                 230                 235                 240
   Leu Pro Gly Pro Gln Gly Pro Pro Gly Phe Gln Gly Leu Gln Gly Thr
                   245                 250                 255
   Val Gly Glu Pro Gly Val Pro Gly Pro Arg Gly Leu Pro Gly Leu Pro
               260                 265                 270
   Gly Val Pro Gly Met Pro Gly Pro Lys Gly Pro Pro Gly Pro Pro Gly
           275                 280                 285
   Pro Ser Gly Ala Val Val Pro Leu Ala Leu Gln Asn Glu Pro Thr Pro
       290                 295                 300
   Ala Pro Glu Asp Asn Gly Cys Pro Pro His Trp Lys Asn Phe Thr Asp
   305                 310                 315                 320
   Lys Cys Tyr Tyr Phe Ser Val Glu Lys Glu Ile Phe Glu Asp Ala Lys
                   325                 330                 335
   Leu Phe Cys Glu Asp Lys Ser Ser His Leu Val Phe Ile Asn Thr Arg
               340                 345                 350
   Glu Glu Gln Gln Trp Ile Lys Lys Gln Met Val Gly Arg Glu Ser His
           355                 360                 365
   Trp Ile Gly Leu Thr Asp Ser Glu Arg Glu Asn Glu Trp Lys Trp Leu
       370                 375                 380
   Asp Gly Thr Ser Pro Asp Tyr Lys Asn Trp Lys Ala Gly Gln Pro Asp
   385                 390                 395                 400
   Asn Trp Gly His Gly His Gly Pro Gly Glu Asp Cys Ala Gly Leu Ile
                   405                 410                 415
   Tyr Ala Gly Gln Trp Asn Asp Phe Gln Cys Glu Asp Val Asn Asn Phe
               420                 425                 430
   Ile Cys Glu Lys Asp Arg Glu Thr Val Leu Ser Ser Ala Leu
           435                 440                 445
<210> 5
   <211> 457
   <212> PRT
   <213> Homo Sapiens
<400> 5
   Met Asn Ser Gln Leu Asn Ser Phe Thr Gly Gln Met Glu Asn Ile Thr
    1               5                  10                  15
   Thr Ile Ser Gln Ala Asn Glu Gln Asn Leu Lys Asp Leu Gln Asp Leu
               20                  25                  30
   His Lys Asp Ala Glu Asn Arg Thr Ala Ile Lys Phe Asn Gln Leu Glu
           35                  40                  45
   Glu Arg Phe Gln Leu Phe Glu Thr Asp Ile Val Asn Ile Ile Ser Asn
       50                  55                  60
   Ile Ser Tyr Thr Ala His His Leu Arg Thr Leu Thr Ser Asn Leu Asn
   65                  70                  75                  80
   Glu Val Arg Thr Thr Cys Thr Asp Thr Leu Thr Lys His Thr Asp Asp
                   85                  90                  95
   Leu Thr Ser Leu Asn Asn Thr Leu Ala Asn Ile Arg Leu Asp Ser Val
               100                 105                 110
   Ser Leu Arg Met Gln Gln Asp Leu Met Arg Ser Arg Leu Asp Thr Glu
           115                 120                 125
   Val Ala Asn Leu Ser Val Ile Met Glu Glu Met Lys Leu Val Asp Ser
       130                 135                 140
   Lys His Gly Gln Leu Ile Lys Asn Phe Thr Ile Leu Gln Gly Pro Pro
   145                 150                 155                 160
   Gly Pro Arg Gly Pro Arg Gly Asp Arg Gly Ser Gln Gly Pro Pro Gly
                   165                 170                 175
   Pro Thr Gly Asn Lys Gly Gln Lys Gly Glu Lys Gly Glu Pro Gly Pro
               180                 185                 190
   Pro Gly Pro Ala Gly Glu Arg Gly Pro Ile Gly Pro Ala Gly Pro Pro
           195                 200                 205
   Gly Glu Arg Gly Gly Lys Gly Ser Lys Gly Ser Gln Gly Pro Lys Gly
       210                 215                 220
   Ser Arg Gly Ser Pro Gly Lys Pro Gly Pro Gln Gly Pro Ser Gly Asp
   225                 230                 235                 240
   Pro Gly Pro Pro Gly Pro Pro Gly Lys Glu Gly Leu Pro Gly Pro Gln
                   245                 250                 255
   Gly Pro Pro Gly Phe Gln Gly Leu Gln Gly Thr Val Gly Glu Pro Gly
               260                 265                 270
   Val Pro Gly Pro Arg Gly Leu Pro Gly Leu Pro Gly Val Pro Gly Met
           275                 280                 285
   Pro Gly Pro Lys Gly Pro Pro Gly Pro Pro Gly Pro Ser Gly Ala Val
       290                 295                 300
   Val Pro Leu Ala Leu Gln Asn Glu Pro Thr Pro Ala Pro Glu Asp Asn
   305                 310                 315                 320
   Gly Cys Pro Pro His Trp Lys Asn Phe Thr Asp Lys Cys Tyr Tyr Phe
                   325                 330                 335
   Ser Val Glu Lys Glu Ile Phe Glu Asp Ala Lys Leu Phe Cys Glu Asp
               340                 345                 350
   Lys Ser Ser His Leu Val Phe Ile Asn Thr Arg Glu Glu Gln Gln Trp
           355                 360                 365
   Ile Lys Lys Gln Met Val Gly Arg Glu Ser His Trp Ile Gly Leu Thr
       370                 375                 380
   Asp Ser Glu Arg Glu Asn Glu Trp Lys Trp Leu Asp Gly Thr Ser Pro
   385                 390                 395                 400
   Asp Tyr Lys Asn Trp Lys Ala Gly Gln Pro Asp Asn Trp Gly His Gly
                   405                 410                 415
   His Gly Pro Gly Glu Asp Cys Ala Gly Leu Ile Tyr Ala Gly Gln Trp
               420                 425                 430
   Asn Asp Phe Gln Cys Glu Asp Val Asn Asn Phe Ile Cys Glu Lys Asp
           435                 440                 445
   Arg Glu Thr Val Leu Ser Ser Ala Leu
       450                 455
<210> 6
   <211> 539
   <212> PRT
   <213> Homo Sapiens
<400> 6
   Met Asn Leu Asn Asn Leu Asn Leu Thr Gln Val Gln Gln Arg Asn Leu
    1               5                  10                  15
   Ile Thr Asn Leu Gln Arg Ser Val Asp Asp Thr Ser Gln Ala Ile Gln
               20                  25                  30
   Arg Ile Lys Asn Asp Phe Gln Asn Leu Gln Gln Val Phe Leu Gln Ala
           35                  40                  45
   Lys Lys Asp Thr Asp Trp Leu Lys Glu Lys Val Gln Ser Leu Gln Thr
       50                  55                  60
   Leu Ala Ala Asn Asn Ser Ala Leu Ala Lys Ala Asn Asn Asp Thr Leu
   65                  70                  75                  80
   Glu Asp Met Asn Ser Gln Leu Asn Ser Phe Thr Gly Gln Met Glu Asn
                   85                  90                  95
   Ile Thr Thr Ile Ser Gln Ala Asn Glu Gln Asn Leu Lys Asp Leu Gln
               100                 105                 110
   Asp Leu His Lys Asp Ala Glu Asn Arg Thr Ala Ile Lys Phe Asn Gln
           115                 120                 125
   Leu Glu Glu Arg Phe Gln Leu Phe Glu Thr Asp Ile Val Asn Ile Ile
       130                 135                 140
   Ser Asn Ile Ser Tyr Thr Ala His His Leu Arg Thr Leu Thr Ser Asn
   145                 150                 155                 160
   Leu Asn Glu Val Arg Thr Thr Cys Thr Asp Thr Leu Thr Lys His Thr
                   165                 170                 175
   Asp Asp Leu Thr Ser Leu Asn Asn Thr Leu Ala Asn Ile Arg Leu Asp
               180                 185                 190
   Ser Val Ser Leu Arg Met Gln Gln Asp Leu Met Arg Ser Arg Leu Asp
           195                 200                 205
   Thr Glu Val Ala Asn Leu Ser Val Ile Met Glu Glu Met Lys Leu Val
       210                 215                 220
   Asp Ser Lys His Gly Gln Leu Ile Lys Asn Phe Thr Ile Leu Gln Gly
   225 230 235 240
   Pro Pro Gly Pro Arg Gly Pro Arg Gly Asp Arg Gly Ser Gln Gly Pro
   245 250 255
   Pro Gly Pro Thr Gly Asn Lys Gly Gln Lys Gly Glu Lys Gly Glu Pro
   260 265 270
   Gly Pro Pro Gly Pro Ala Gly Glu Arg Gly Pro Ile Gly Pro Ala Gly
   275 280 285
   Pro Pro Gly Glu Arg Gly Gly Lys Gly Ser Lys Gly Ser Gln Gly Pro
   290 295 300
   Lys Gly Ser Arg Gly Ser Pro Gly Lys Pro Gly Pro Gln Gly Pro Ser
   305 310 315 320
   Gly Asp Pro Gly Pro Pro Gly Pro Pro Gly Lys Glu Gly Leu Pro Gly
   325 330 335
   Pro Gln Gly Pro Pro Gly Phe Gln Gly Leu Gln Gly Thr Val Gly Glu
   340 345 350
   Pro Gly Val Pro Gly Pro Arg Gly Leu Pro Gly Leu Pro Gly Val Pro
   355 360 365
   Gly Met Pro Gly Pro Lys Gly Pro Pro Gly Pro Pro Gly Pro Ser Gly
   370 375 380
   Ala Val Val Pro Leu Ala Leu Gln Asn Glu Pro Thr Pro Ala Pro Glu
   385 390 395 400
   Asp Asn Gly Cys Pro Pro His Trp Lys Asn Phe Thr Asp Lys Cys Tyr
   405 410 415
   Tyr Phe Ser Val Glu Lys Glu Ile Phe Glu Asp Ala Lys Leu Phe Cys
   420 425 430
   Glu Asp Lys Ser Ser His Leu Val Phe Ile Asn Thr Arg Glu Glu Gln
   435 440 445
   Gln Trp Ile Lys Lys Gln Met Val Gly Arg Glu Ser His Trp Ile Gly
   450 455 460
   Leu Thr Asp Ser Glu Arg Glu Asn Glu Trp Lys Trp Leu Asp Gly Thr
   465 470 475 480
   Ser Pro Asp Tyr Lys Asn Trp Lys Ala Gly Gln Pro Asp Asn Trp Gly
   485 490 495
   His Gly His Gly Pro Gly Glu Asp Cys Ala Gly Leu Ile Tyr Ala Gly
   500 505 510
   Gln Trp Asn Asp Phe Gln Cys Glu Asp Val Asn Asn Phe Ile Cys Glu
   515 520 525
   Lys Asp Arg Glu Thr Val Leu Ser Ser Ala Leu
   530 535
<210> 7
   <211> 547
   <212> PRT
   <213> Homo Sapiens
<400> 7
   Met Tyr Ser His Asn Val Val Ile Met Asn Leu Asn Asn Leu Asn Leu
    1               5                  10                  15
   Thr Gln Val Gln Gln Arg Asn Leu Ile Thr Asn Leu Gln Arg Ser Val
               20                  25                  30
   Asp Asp Thr Ser Gln Ala Ile Gln Arg Ile Lys Asn Asp Phe Gln Asn
           35                  40                  45
   Leu Gln Gln Val Phe Leu Gln Ala Lys Lys Asp Thr Asp Trp Leu Lys
       50                  55                  60
   Glu Lys Val Gln Ser Leu Gln Thr Leu Ala Ala Asn Asn Ser Ala Leu
   65                  70                  75                  80
   Ala Lys Ala Asn Asn Asp Thr Leu Glu Asp Met Asn Ser Gln Leu Asn
                   85                  90                  95
   Ser Phe Thr Gly Gln Met Glu Asn Ile Thr Thr Ile Ser Gln Ala Asn
               100                 105                 110
   Glu Gln Asn Leu Lys Asp Leu Gln Asp Leu His Lys Asp Ala Glu Asn
           115                 120                 125
   Arg Thr Ala Ile Lys Phe Asn Gln Leu Glu Glu Arg Phe Gln Leu Phe
           130                 135                 140
   Glu Thr Asp Ile Val Asn Ile Ile Ser Asn Ile Ser Tyr Thr Ala His
   145                 150                 155                 160
   His Leu Arg Thr Leu Thr Ser Asn Leu Asn Glu Val Arg Thr Thr Cys
                   165                 170                 175
   Thr Asp Thr Leu Thr Lys His Thr Asp Asp Leu Thr Ser Leu Asn Asn
               180                 185                 190
   Thr Leu Ala Asn Ile Arg Leu Asp Ser Val Ser Leu Arg Met Gln Gln
           195                 200                 205
   Asp Leu Met Arg Ser Arg Leu Asp Thr Glu Val Ala Asn Leu Ser Val
       210                 215                 220
   Ile Met Glu Glu Met Lys Leu Val Asp Ser Lys His Gly Gln Leu Ile
   225                 230                 235                 240
   Lys Asn Phe Thr Ile Leu Gln Gly Pro Pro Gly Pro Arg Gly Pro Arg
                   245                 250                 255
   Gly Asp Arg Gly Ser Gln Gly Pro Pro Gly Pro Thr Gly Asn Lys Gly
               260                 265                 270
   Gln Lys Gly Glu Lys Gly Glu Pro Gly Pro Pro Gly Pro Ala Gly Glu
           275                 280                 285
   Arg Gly Pro Ile Gly Pro Ala Gly Pro Pro Gly Glu Arg Gly Gly Lys
       290                 295                 300
   Gly Ser Lys Gly Ser Gln Gly Pro Lys Gly Ser Arg Gly Ser Pro Gly
   305                 310                 315                 320
   Lys Pro Gly Pro Gln Gly Pro Ser Gly Asp Pro Gly Pro Pro Gly Pro
                   325                 330                 335
   Pro Gly Lys Glu Gly Leu Pro Gly Pro Gln Gly Pro Pro Gly Phe Gln
               340                 345                 350
   Gly Leu Gln Gly Thr Val Gly Glu Pro Gly Val Pro Gly Pro Arg Gly
           355                 360                 365
   Leu Pro Gly Leu Pro Gly Val Pro Gly Met Pro Gly Pro Lys Gly Pro
       370                 375                 380
   Pro Gly Pro Pro Gly Pro Ser Gly Ala Val Val Pro Leu Ala Leu Gln
   385                 390                 395                 400
   Asn Glu Pro Thr Pro Ala Pro Glu Asp Asn Gly Cys Pro Pro His Trp
                   405                 410                 415
   Lys Asn Phe Thr Asp Lys Cys Tyr Tyr Phe Ser Val Glu Lys Glu Ile
               420                 425                 430
   Phe Glu Asp Ala Lys Leu Phe Cys Glu Asp Lys Ser Ser His Leu Val
           435                 440                 445
   Phe Ile Asn Thr Arg Glu Glu Gln Gln Trp Ile Lys Lys Gln Met Val
       450                 455                 460
   Gly Arg Glu Ser His Trp Ile Gly Leu Thr Asp Ser Glu Arg Glu Asn
   465                 470                 475                 480
   Glu Trp Lys Trp Leu Asp Gly Thr Ser Pro Asp Tyr Lys Asn Trp Lys
                   485                 490                 495
   Ala Gly Gln Pro Asp Asn Trp Gly His Gly His Gly Pro Gly Glu Asp
               500                 505                 510
   Cys Ala Gly Leu Ile Tyr Ala Gly Gln Trp Asn Asp Phe Gln Cys Glu
           515                 520                 525
   Asp Val Asn Asn Phe Ile Cys Glu Lys Asp Arg Glu Thr Val Leu Ser
       530                 535                 540
   Ser Ala Leu
   545
<210> 8
   <211> 24
   <212> DNA
   <213> Artificial Sequence
<220>
   <223> M13 Universal Primer
<400> 8
   cgacgttgta aaacgacggc cagt 24
<210> 9
   <211> 17
   <212> DNA
   <213> Artificial Sequence
<220>
   <223> M13 Reverse Primer
<400> 9
   caggaaaca gctatgac 17
<210> 10
   <211> 21
   <212> DNA
   <213> Artificial Sequence
<220>
   <223> Reverse Primer
<400> 10
   caatctgatg agaaggtgat g 21
<210> 11
   <211> 21
   <212> DNA
   <213> Artificial Sequence
<220>
   <223> Forward Primer
<400> 11
   acgaggggct ggatgggaca t 21
<210> 12
   <211> 21
   <212> DNA
   <213> Artificial Sequence
<220>
   <223> Primer
<400> 12
   cgtgaaaatg aatggaagtg g 21
<210> 13
   <211> 21
   <212> DNA
   <213> Artificial Sequence
<220>
   <223> Primer for Screening a Novel Collectin.
<400> 13
   ttttatccat tgctgttcct c 21
<210> 14
   <211> 21
   <212> DNA
   <213> Artificial Sequence
<220>
   <223> Primer for Sequencing a Novel Collectin.
<400> 14
   ctggcagtcc ccgaggtcca g 21
<210> 15
   <211> 21
   <212> DNA
   <213> Artificial Sequence
<220>
   <223> Primer
<400> 15
   gctggtcccc ccggagagcg t 21
<210> 16
   <211> 21
   <212> DNA
   <213> Artificial Sequence
<220>
   <223> 1RC2 Primer
<400> 16
   caaggtacgc cacagcgtat g 21
<210> 17
   <211> 20
   <212> DNA
   <213> Artificial Sequence
<220>
   <223> TGP1 Primer
<400> 17
   tcttcagttt ccctaatccc 20
<210> 18
   <211> 21
   <212> DNA
   <213> Artificial Sequence
<220>
   <223> 2RC2 Primer
<400> 18
   gtacgccaca gcgtatgatg c 21
<210> 19
   <211> 21
   <212> DNA
   <213> Artificial Sequence
<220>
   <223> TGP2 Primer
<400> 19
   cattcttgac aaacttcata g 21
<210> 20
   <211> 2024
   <212> DNA
   <213> Homo Sapiens
<220>
   <221> CDS
   <222> (670)..(1695)
<400> 20
   gtcacgaatc tgcagcaaga taccagcgtg ctccagggca atctgcagaa ccaaatgtat    60
   tctcataatg tggtcatcat gaacctcaac aacctgaacc tgacccaggt gcagcagagg   120
   aacctcatca cgaatctgca gcggtctgtg gatgacacaa gccaggctat ccagcgaatc   180
   aagaacgact ttcaaaatct gcagcaggtt tttcttcaag ccaagaagga cacggattgg   240
   ctgaaggaga aagtgcagag cttgcagacg ctggctgcca acaactctgc gttggccaaa   300
   gccaacaacg acaccctgga ggatatgaac agccagctca actcattcac aggtcagatg   360
   gagaacatca ccactatctc tcaagccaac gagcagaacc tgaaagacct gcaggactta   420
   cacaaagatg cagagaatag aacagccatc aagttcaacc aactggagga acgcttccag   480
   ctctttgaga cggatattgt gaacatcatt agcaatatca gttacacagc ccaccacctg   540
   cggacgctga ccagcaatct aaatgaagtc aggaccactt gcacagatac ccttaccaaa   600
   cacacagatg atctgacctc cttgaataat accctggcca acatccgttt ggattctgtt   660
   tctctcagg atg caa caa gat ttg atg agg tcg agg tta gac act gaa gta   711
             Met Gln Gln Asp Leu Met Arg Ser Arg Leu Asp Thr Glu Val
                 1               5                   10
   gcc aac tta tca gtg att atg gaa gaa atg aag cta gta gac tcc aag     759
   Ala Asn Leu Ser Val Ile Met Glu Glu Met Lys Leu Val Asp Ser Lys
    15                  20                  25                  30
   cat ggt cag ctc atc aag aat ttt aca ata cta caa ggt cca ccg ggc     807
   His Gly Gln Leu Ile Lys Asn Phe Thr Ile Leu Gln Gly Pro Pro Gly
                   35                  40                  45
   ccc agg ggt cca aga ggt gac aga gga tcc cag gga ccc cct ggc cca     855
   Pro Arg Gly Pro Arg Gly Asp Arg Gly Ser Gln Gly Pro Pro Gly Pro
               50                  55                  60
   act ggc aac aag gga cag aaa gga gag aag ggg gag cct gga cca cct     903
   Thr Gly Asn Lys Gly Gln Lys Gly Glu Lys Gly Glu Pro Gly Pro Pro
           65                  70                  75
   ggc cct gcg ggt gag aga ggc cca att gga cca gct ggt ccc ccc gga     951
   Gly Pro Ala Gly Glu Arg Gly Pro Ile Gly Pro Ala Gly Pro Pro Gly
       80                  85                  90
   gag cgt ggc ggc aaa gga tct aaa ggc tcc cag ggc ccc aaa ggc tcc     999
   Glu Arg Gly Gly Lys Gly Ser Lys Gly Ser Gln Gly Pro Lys Gly Ser
    95                 100                 105                 110
   cgt ggt tcc cct ggg aag ccc ggc cct cag ggc ccc agt ggg gac cca    1047
   Arg Gly Ser Pro Gly Lys Pro Gly Pro Gln Gly Pro Ser Gly Asp Pro
                   115                 120                 125
   ggc ccc ccg ggc cca cca ggc aaa gag gga ctc ccc ggc cct cag ggc    1095
   Gly Pro Pro Gly Pro Pro Gly Lys Glu Gly Leu Pro Gly Pro Gln Gly
               130                 135                 140
   cct cct ggc ttc cag gga ctt cag ggc acc gtt ggg gag cct ggg gtg    1143
   Pro Pro Gly Phe Gln Gly Leu Gln Gly Thr Val Gly Glu Pro Gly Val
           145                 150                 155
   cct gga cct cgg gga ctg cca ggc ttg cct ggg gta cca ggc atg cca    1191
   Pro Gly Pro Arg Gly Leu Pro Gly Leu Pro Gly Val Pro Gly Met Pro
       160                 165                 170
   ggc ccc aag ggc ccc ccc ggc cct cct ggc cca tca gga gcg gtg gtg    1239
   Gly Pro Lys Gly Pro Pro Gly Pro Pro Gly Pro Ser Gly Ala Val Val
   175                 180                 185                 190
   ccc ctg gcc ctg cag aat gag cca acc ccg gca ccg gag gac aat ggc    1287
   Pro Leu Ala Leu Gln Asn Glu Pro Thr Pro Ala Pro Glu Asp Asn Gly
                   195                 200                 205
   tgc ccg cct cac tgg aag aac ttc aca gac aaa tgc tac tat ttt tca    1335
   Cys Pro Pro His Trp Lys Asn Phe Thr Asp Lys Cys Tyr Tyr Phe Ser
               210                 215                 220
   gtt gag aaa gaa att ttt gag gat gca aag ctt ttc tgt gaa gac aag    1383
   Val Glu Lys Glu Ile Phe Glu Asp Ala Lys Leu Phe Cys Glu Asp Lys
           225                 230                 235
   tct tca cat ctt gtt ttc ata aac act aga gag gaa cag caa tgg ata    1431
   Ser Ser His Leu Val Phe Ile Asn Thr Arg Glu Glu Gln Gln Trp Ile
       240                 245                 250
   aaa aaa cag atg gta ggg aga gag agc cac tgg atc ggc ctc aca gac    1479
   Lys Lys Gln Met Val Gly Arg Glu Ser His Trp Ile Gly Leu Thr Asp
   255                 260                 265                 270
   tca gag cgt gaa aat gaa tgg aag tgg ctg gat ggg aca tct cca gac    1527
   Ser Glu Arg Glu Asn Glu Trp Lys Trp Leu Asp Gly Thr Ser Pro Asp
                       275                   280                   285
   tac aaa aat tgg aaa gct gga cag ccg gat aac tgg ggt cat ggc cat     1575
   Tyr Lys Asn Trp Lys Ala Gly Gln Pro Asp Asn Trp Gly His Gly His
                   290                   295                   300
   ggg cca gga gaa gac tgt gct ggg ttg att tat gct ggg cag tgg aac     1623
   Gly Pro Gly Glu Asp Cys Ala Gly Leu Ile Tyr Ala Gly Gln Trp Asn
               305                   310                   315
   gat ttc caa tgt gaa gac gtc aat aac ttc att tgc gaa aaa gac agg     1671
   Asp Phe Gln Cys Glu Asp Val Asn Asn Phe Ile Cys Glu Lys Asp Arg
           320                   325                   330
   gag aca gta ctg tca tct gca tta taacggactg tgatgggatc acatgagcaa    1725
   Glu Thr Val Leu Ser Ser Ala Leu
   335                 340
   attttcagct ctcaaaggca aaggacactc ctttctaatt gcatcacctt ctcatcagat   1785
   tgaaaaaaaa aaaagcactg aaaaccaatt actgaaaaaa aattgacagc tagtgttttt   1845
   taccatccgt cattacccaa agacttggga actaaaatgt tccccagggt gatatgctga   1905
   ttttcattgt gcacatggac tgaatcacat agattctcct ccgtcagtaa ccgtgcgatt   1965
   atacaaatta tgtcttccaa agtatggaac actccaatca gaaaaaggtt atcatcccg    2024
<210> 21
   <211> 22
   <212> DNA
   <213> Artificial Sequence
<220>
   <223> Primer
<400> 21
   gaagacaagt cttcaactct tg 22
<210> 22
   <211> 22
   <212> DNA
   <213> Artificial Sequence
<220>
   <223> Primer
<400> 22
   ctctgagtct gtgaggccga tc 22
<210> 23
   <211> 111
   <212> DNA
   <213> Artificial Sequence
<220>
   <223> Probe
<400> 23
   gaagacaagt cttcacatct tgttttcata aacactagag aggaacagca atggataaaa    60
   aaacagatgg tagggagaga gagccactgg atcggcctca cagactcaga g            111
<210> 24
   <211> 22
   <212> DNA
   <213> Artificial Sequence
<220>
   <223> Forward Primer
<400> 24
   gtgcccctgg ccctgcagaa tg 22
<210> 25
   <211> 26
   <212> DNA
   <213> Artificial Sequence
<220>
   <223> Reverse Primer
<400> 25
   gcatatcacc ctggggaaca ttttag 26
<210> 26
   <211> 39
   <212> DNA
   <213> Artificial Sequence
<220>
   <223> Sense Primer
<400> 26
   aaggaaaaaa gcggccgcat gcaacaagat ttgatgagg 39
<210> 27
   <211> 29
   <212> DNA
   <213> Artificial Sequence
<220>
   <223> Reverse Primer
<400> 27
   gctctagatt ataatgcaga tgacagtac 29
<210> 28
   <211> 248
   <212> PRT
   <213> Homo Sapiens
<220>
   <223> Amino Acid Sequence of Human MBP
<400> 28
   Met Ser Leu Phe Pro Ser Leu Pro Leu Leu Leu Leu Ser Met Val Ala
    1               5                  10                  15
   Ala Ser Tyr Ser Glu Thr Val Thr Cys Glu Asp Ala Gln Lys Thr Cys
               20                  25                  30
   Pro Ala Val Ile Ala Cys Ser Ser Pro Gly Ile Asn Gly Phe Pro Gly
           35                  40                  45
   Lys Asp Gly Arg Asp Gly Thr Lys Gly Glu Lys Gly Glu Pro Gly Gln
       50                  55                  60
   Gly Leu Arg Gly Leu Gln Gly Pro Pro Gly Lys Leu Gly Pro Pro Gly
   65                  70                  75                  80
   Asn Pro Gly Pro Ser Gly Ser Pro Gly Pro Lys Gly Gln Lys Gly Asp
                   85                  90                  95
   Pro Gly Lys Ser Pro Asp Gly Asp Ser Ser Leu Ala Ala Ser Glu Arg
               100                 105                 110
   Lys Ala Leu Gln Thr Glu Met Ala Arg Ile Lys Lys Trp Leu Thr Phe
           115                 120                 125
   Ser Leu Gly Lys Gln Val Gly Asn Lys Phe Phe Leu Thr Asn Gly Glu
       130                 135                 140
   Ile Met Thr Phe Glu Lys Val Lys Ala Leu Cys Val Lys Phe Gln Ala
   145                 150                 155                 160
   Ser Val Ala Thr Pro Arg Asn Ala Ala Glu Asn Gly Ala Ile Gln Asn
                   165                 170                 175
   Leu Ile Lys Glu Glu Ala Phe Leu Gly Ile Thr Asp Glu Lys Thr Glu
               180                 185                 190
   Gly Gln Phe Val Asp Leu Thr Gly Asn Arg Leu Thr Tyr Thr Asn Trp
           195                 200                 205
   Asn Glu Gly Glu Pro Asn Asn Ala Gly Ser Asp Glu Asp Cys Val Leu
       210                 215                 220
   Leu Leu Lys Asn Gly Gln Trp Asn Asp Val Pro Cys Ser Thr Ser His
   225                 230                 235                 240
   Leu Ala Val Cys Glu Phe Pro Ile
                   245
<210> 29
   <211> 248
   <212> PRT
   <213> Homo Sapiens
<220>
   <223> Amino Acid Sequence of Human SP-A
<400> 29
<210> 30
   <211> 375
   <212> PRT
   <213> Homo Sapiens
<220>
   <223> Amino Acid Sequence of Human SP-D
<400> 30
   Met Leu Leu Phe Leu Leu Ser Ala Leu Val Leu Leu Thr Gln Pro Leu
    1               5                  10                  15
   Gly Tyr Leu Glu Ala Glu Met Lys Thr Tyr Ser His Arg Thr Met Pro
               20                  25                  30
   Ser Ala Cys Thr Leu Val Met Cys Ser Ser Val Glu Ser Gly Leu Pro
            35                  40                  45
   Gly Arg Asp Gly Arg Asp Gly Arg Glu Gly Pro Arg Gly Glu Lys Gly
        50                  55                  60
   Asp Pro Gly Leu Pro Gly Ala Ala Gly Gln Ala Gly Met Pro Gly Gln
    65                  70                  75                  80
   Ala Gly Pro Val Gly Pro Lys Gly Asp Asn Gly Ser Val Gly Glu Pro
                   85                  90                  95
   Gly Pro Lys Gly Asp Thr Gly Pro Ser Gly Pro Pro Gly Pro Pro Gly
               100                 105                 110
   Val Pro Gly Pro Ala Gly Arg Glu Gly Ala Leu Gly Lys Gln Gly Asn
           115                 120                 125
   Ile Gly Pro Gln Gly Lys Pro Gly Pro Lys Gly Glu Ala Gly Pro Lys
       130                 135                 140
   Gly Glu Val Gly Ala Pro Gly Met Gln Gly Ser Ala Gly Ala Arg Gly
   145                 150                 155                 160
   Leu Ala Gly Pro Lys Gly Glu Arg Gly Val Pro Gly Glu Arg Gly Val
                   165                 170                 175
   Pro Gly Asn Thr Gly Ala Ala Gly Ser Ala Gly Ala Met Gly Pro Gln
               180                 185                 190
   Gly Ser Pro Gly Ala Arg Gly Pro Pro Gln Leu Lys Gly Asp Lys Gly
           195                 200                 205
   Ile Pro Gly Asp Lys Gly Ala Lys Gly Glu Ser Gly Leu Pro Asp Val
       210                 215                 220
   Ala Ser Leu Arg Gln Gln Val Glu Ala Leu Gln Gly Gln Val Gln His
   225                 230                 235                 240
   Leu Gln Ala Ala Phe Ser Gln Tyr Lys Lys Val Glu Leu Phe Pro Asn
                   245                 250                 255
   Gly Gln Ser Val Gly Glu Lys Ile Phe Lys Thr Ala Gly Phe Val Lys
               260                 265                 270
   Pro Phe Thr Glu Ala Gln Leu Leu Cys Thr Gln Ala Gly Gly Gln Leu
           275                 280                 285
   Ala Ser Pro Arg Ser Ala Ala Glu Asn Ala Ala Leu Gln Gln Leu Val
       290                 295                 300
   Val Ala Lys Asn Glu Ala Ala Phe Leu Ser Met Thr Asp Ser Lys Thr
   305                 310                 315                 320
   Glu Gly Lys Phe Thr Tyr Pro Thr Gly Glu Ser Leu Val Tyr Ser Asn
                   325                 330                 335
   Trp Ala Pro Gly Glu Pro Asn Asp Asp Gly Gly Ser Glu Asp Cys Val
               340                 345                 350
   Glu Ile Phe Thr Asn Gly Lys Trp Asn Asp Arg Ala Cys Gly Glu Lys
           355                 360                 365
   Arg Leu Val Val Cys Glu Phe
       370                 375